(19) Europäisches Patentamt
European Patent Office
Office européen des brevets

(11) Numéro de publication : **0 624 582 A1**

(12) **DEMANDE DE BREVET EUROPEEN**

(21) Numéro de dépôt : **94400977.8**

(22) Date de dépôt : **05.05.94**

(51) Int. Cl.[5] : **C07D 319/20,** C07D 405/12, A61K 31/335

(30) Priorité : **07.05.93 FR 9305467**

(43) Date de publication de la demande : **17.11.94 Bulletin 94/46**

(84) Etats contractants désignés : **AT BE CH DE DK ES FR GB GR IE IT LI LU NL PT SE**

(71) Demandeur : **ADIR ET COMPAGNIE
1 rue Carle Hébert
F-92415 Courbevoie Cédex (FR)**

(72) Inventeur : **Guillaumet, Gérald
2, rue Auguste Renoir
F-45100 Orleans (FR)**
Inventeur : **Coudert, Gérard
5, rue Paul Landowski
F-45100 Orleans (FR)**
Inventeur : **Thiery, Valérie
146, rue du Maréchal Foch
Clery St Andre (FR)**
Inventeur : **Adam, Gérard
9, Clos du Mesnil,
Route du Pecq
F-78600 Le Mesnil le Roi (FR)**
Inventeur : **Bizot-Espiard, Jean-Guy
190, rue de Vaugirard
F-75015 Paris (FR)**
Inventeur : **Pfeiffer, Bruno
6, rue Jean Thomas
F-95600 Eaubonne (FR)**
Inventeur : **Renard, Pierre
50, av. de Villeneuve l'Etang
F-78000 Versailles (FR)**

(54) **Benzodioxines substituées, leur procédé de préparation et les compositions pharmaceutiques les contenant.**

(57) Composés de formule générale (I) :

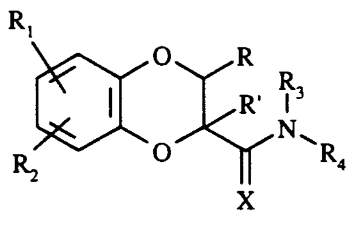

(I)

dans laquelle :
— X représente O, S ou $H_2$,
— R et R' représentent chacun l'hydrogène, ou forment ensemble une liaison,
— $R_1$, $R_2$, $R_3$ et $R_4$ sont tels que définis dans la description,
Ces composés trouvent leur application en thérapeutique dans le traitement ou la prévention des affections où interviennent des processus oxydatifs.

EP 0 624 582 A1

La présente invention concerne de nouvelles benzodioxines substituées, leur procédé de préparation et les compositions pharmaceutiques qui les contiennent.

La littérature fournit de nombreux exemples de 1,4-benzodioxines substituées utilisées dans des domaines variés.

Le brevet EP-A-515941 présente des 6-(pyrrol-2-yl)benzodioxanes utilisées comme pesticides. Le brevet EP-A-520674 décrit des dérivés de N-amino-phénoxy-alkyl-2,3-dihydro-1,4-benzodioxin-2-méthanamine qui sont des agonistes de dopamine.

Dans leurs travaux, (Farmatsiya (Sofia), 27, (5), 1-5, (1977)), Velichkof *et al.* exposent une voie de synthèse de 1,4-benzodioxan-2,3-dicarboxamides N-substituées.

La demanderesse a découvert que certaines benzodioxines substituées en position 2 par un groupement carboxamide, thiocarboxamide ou méthylène amine sont douées de propriétés antioxydantes particulièrement intéressantes sur le plan pharmacologique.

La demanderesse a découvert de nouvelles benzodioxines substituées ainsi que leurs propriétés pharmacologiques très intéressantes de par leur pouvoir inhibiteur vis-à-vis de l'oxydation des lipides et en particulier des lipoprotéines.

Plus spécifiquement, l'invention concerne de nouvelles benzodioxines substituées de formule générale (I) :

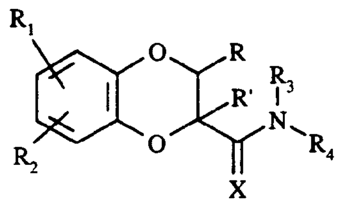

(I)

dans laquelle :

- R et R' représentent chacun un atome d'hydrogène, cas des 2,3-dihydrobenzo-dioxines, ou forment ensemble une liaison, cas des benzodioxines,
- $R_1$ et $R_2$ identiques ou différents sont choisis indépendamment l'un de l'autre parmi un atome d'hydrogène, un radical alkyle, contenant de 1 à 6 atomes de carbone en chaîne droite ou ramifiée, un radical hydroxy, un radical alkoxy contenant de 1 à 5 atomes de carbone en chaîne droite ou ramifiée, un groupement :

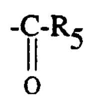 , et un groupement 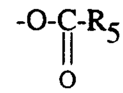 ,

dans lesquels $R_5$ est choisi parmi un radical alkyle contenant de 1 à 5 atomes de carbone en chaîne droite ou ramifiée, un radical aryle éventuellement substitué choisi parmi les radicaux phényle et naphtyle, un radical arylalkyle, éventuellement substitué, choisi parmi les radicaux phényle et naphtyle fixés sur une chaîne alkyle comportant de 1 à 4 atomes de carbone, un radical hétéroaryle, éventuellement substitué, choisi parmi les radicaux pyridyle, pyrrolyle, pyrazinyle, pyridazinyle, pyrimidinyle, quinolyle, quinazolinyle et indolyle, et un radical hétéroarylalkyle, éventuellement substitué, choisi parmi les radicaux hétéroaryles ci-dessus définis fixés sur une chaîne alkyle comportant de 1 à 4 atomes de carbone,
- $R_3$ et $R_4$, identiques ou différents, sont choisis indépendamment l'un de l'autre parmi un atome d'hydrogène, un radical alkyle contenant de 1 à 5 atomes de carbone en chaîne droite ou ramifiée et éventuellement substitué, un radical aryle, arylaikyle, hétéroaryle et hétéroarylalkyle, chacun pouvant être éventuellement substitué par une ou plusieurs entités chimiques choisies parmi les radicaux définis pour $R_1$ ou $R_2$,

  ou $R_3$ et $R_4$ forment ensemble avec l'atome d'azote auquel ils sont reliés un hétérocycle de formule (I') :

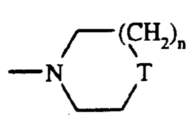

(I')

dans laquelle,
- n est choisi parmi 0, 1, 2 et 3,
- T est choisi parmi l'oxygène, le groupement

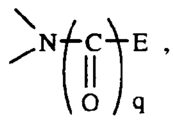

- le groupement

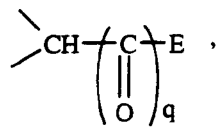

- et le groupement

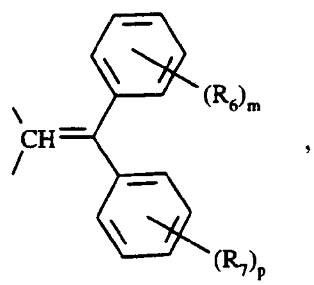

dans lequel :
m et p identiques ou différents prennent indépendamment les valeurs 0, 1, 2, 3, 4 ou 5,
$R_6$ et $R_7$ identiques ou différents, indépendamment l'un de l'autre, sont choisis parmi un halogène, un groupement hydroxy, alkyle linéaire ou ramifié comprenant de 1 à 5 atomes de carbone, un radical alkoxy, un radical halogénoalkyle et un radical polyhalogénoalkyle
- q est choisi parmi 0 et 1,
- E est choisi parmi un atome d'hydrogène, un radical alkyle contenant de 1 à 5 atomes de carbone en chaîne droite ou ramifiée, un radical aryle, arylalkyle, hétéroaryle, hétéroarylalkyle, tels que définis précédemment, chacun éventuellement substitué par un ou plusieurs radicaux $R_6$ tel que définis précédemment, et le groupement :

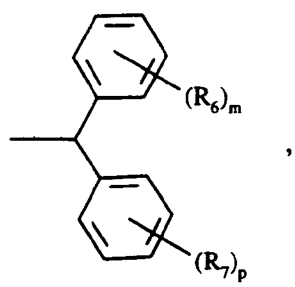

dans lequel $R_6$, $R_7$, m et p sont tels que définis précédemment,
avec la restriction lorsque X = 0 et $R_1$ = $R_2$ = H alors T ne peut représenter un radical $CH_2$ ou un radical quinazolin-2-ylamino,
- et X est choisi parmi O, S et $H_2$,

étant entendu que, sauf indication contraire, le terme "éventuellement substitué" indique une substitution éventuelle par un ou plusieurs radicaux choisis parmi les radicaux hydroxy, nitro, cyano, alkyles, alkoxy, acyles, halogénoalkyles, polyhalogénoalkyles, amino, alkylamino et dialkylamino, les chaînes alkyles des groupements alkyles, alkoxy, acyles, halogénoalkyles, polyhalogénoalkyles, alkylamino et dialkylamino comportant

de 1 à 5 atomes de carbone en chaîne droite ou ramifiée,
avec les restrictions suivantes :

- lorsque R et R' représentent chacun l'hydrogène, alors X ne peut représenter $H_2$,
- lorsque R et R' représentent chacun l'hydrogène et X représente l'oxygène, alors
  - soit $R_1$ et $R_2$ sont chacun différents de l'hydrogène,
  - soit $R_1$ est différent de l'hydrogène et $R_3$ et $R_4$ forment ensemble, avec l'atome d'azote auquel ils sont reliés, un hétérocycle tel que défini précédemment,

leurs éventuels stéréoisomères, N-oxydes et sels d'addition à un acide ou à une base, pharmaceutiquement acceptables.

Parmi les acides pharmaceutiquement acceptables que l'on peut utiliser pour former un sel d'addition avec les composés de l'invention, on peut citer à titre d'exemples non limitatifs, les acides chlorhydrique, phosphorique, sulfurique, tartrique, citrique, maléique, fumarique, alkylsulfonique, arylsulfonique et camphorique.

Parmi les bases pharmaceutiquement acceptables que l'on peut utiliser pour former un sel d'addition avec les composés de l'invention, on peut citer, à titre d'exemples non limitatifs, l'hydroxyde de sodium ou de potassium, la diéthylamine, la triéthylamine, l'éthanolamine, la diéthanolamine, l'arginine et la lysine.

L'invention s'étend également au procédé de préparation des composés de formule (I), caractérisé en ce que l'on effectue une réaction de cyclisation en présence de carbonate alcalin, par exemple le carbonate de potassium, en solvant aprotique, tel que l'acétone, à reflux, à partir de catéchol et de 2,3-dibromopropionate d'alkyle, conduisant au composé de formule (IIa) :

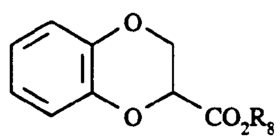

(IIa)

dans laquelle $R_8$ représente un radical alkyle comprenant de 1 à 5 atomes de carbone en chaîne droite ou ramifiée,
qui est éventuellement engagé dans une réaction d'acylation avec un agent d'acylation de formule (III) :

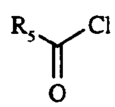

(III)

dans laquelle $R_5$ est tel que défini précédemment,
avec un acide de Lewis, tel que le chlorure d'aluminium dans le sulfure de carbone à O°C, pour conduire au composé de formule (IIb) :

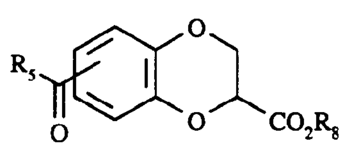

(IIb)

dans laquelle $R_5$ et $R_8$ sont tels que définis précédemment,
qui peut être éventuellement oxydé en diester correspondant de formule (IIc) :

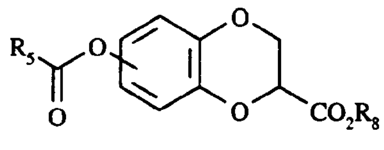

(IIc)

dans laquelle $R_5$ et $R_8$ sont tels que définis précédemment,
au moyen d'un agent d'oxydation, par exemple l'acide métachloroperbenzoïque dans le chlorure de méthylène, puis éventuellement transformé, par action d'un alcoolate alcalin, par exemple l'éthylate de sodium dans le méthanol, en phénol de formule (IId) :

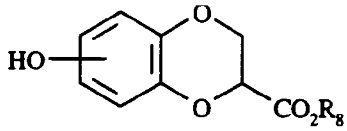

(IId)

dans laquelle $R_8$ est tel que défini précédemment,
les céto-esters de formule (IIb) pouvant d'autre part être hydrogénés, par action d'hydrogène et de palladium, en composé de formule (IIe) :

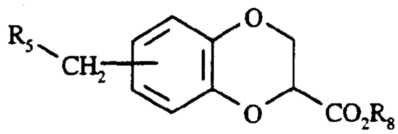

(IIe)

dans laquelle $R_5$ et $R_8$ sont tels que définis précédemment,
le phénol de formule (IId) pouvant être éthérifié par réaction avec un composé halogéné de formule (IV) :

$R_8$-Hal (IV)

dans laquelle Hal est un halogène,
pour conduire au composé de formule (IIf) :

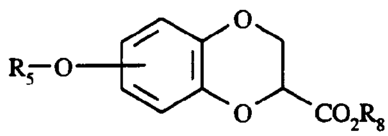

(IIf)

dans laquelle $R_5$ et $R_8$ sont tels que définis précédemment,
ou acylé par un chlorure d'acyle de formule (III) comme défini précédemment pour accéder au diester de formule (IIc) précédemment défini,
les composés de formules (IIa) à (IIf) formant l'ensemble des composés de formule (II) :

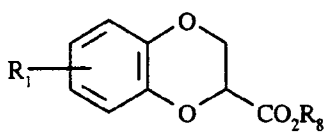

(II)

dans laquelle $R_1$ et $R_8$ sont tels que définis précédemment,
qui peuvent être à nouveau traités comme les composés de formules (IIa) à (IIf) ou en milieu acide par un alcool tertiaire de formule (X) :

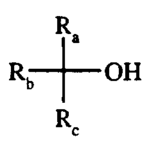

(X)

dans laquelle $R_a$, $R_b$ et $R_c$ représentent indépendamment un radical allyle linéaire ou ramifié contenant de 1 à 3 atomes de carbone,
pour donner les composés de formule (Va) :

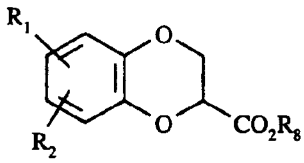

(Va)

dans laquelle $R_1$, $R_2$ et $R_8$ sont tels que définis précédemment,
les benzodioxanes de formules (Va) pouvant être éventuellement traitées par un agent bromant, tel que le N-bromosuccinimide dans le tétrachlorure de carbone, en présence d'un initiateur radicalaire, tel que l'azo-bis-isobutyronitrile pour conduire à l'intermédiaire de formule (VI) :

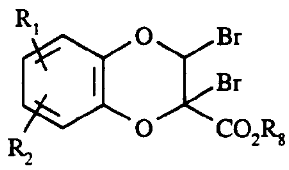

(VI)

dans laquelle $R_1$, $R_2$ et $R_8$ sont tels que définis précédemment,
qui est ensuite soumis à un halogénure alcalin, tel que l'iodure de sodium dans l'acétone, pour fournir les benzodioxines correspondantes de formule (Vb) :

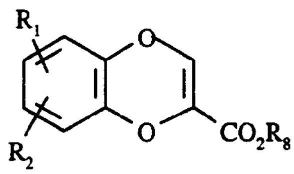

(Vb)

dans laquelle $R_1$, $R_2$ et $R_8$ sont tels que définis précédemment,
les composés de formules (Va) et (Vb) formant l'ensemble des composés de formule (V) :

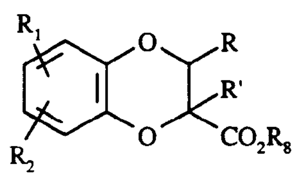

(V)

dans laquelle $R_1$, $R_2$, $R_8$, R et R', sont tels que définis précédemment,
qui sont finalement saponifiés pour conduire aux acides de formule (VII) :

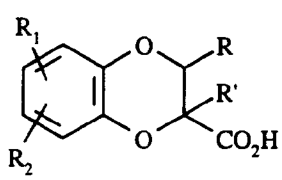

(VII)

dans laquelle R, R', $R_1$ et $R_2$ sont tels que définis précédemment,
qui sont soumis à une réaction de couplage peptidique, en présence de 1-(3-diméthylaminopropyl)-3-éthylcarbodiimide (EDCI) et d'hydroxybenzotriazole (HOBt) dans le diméthylformamide à 0°C, avec une amine de formule (VIII) :

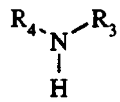

(VIII)

dans laquelle $R_3$ et $R_4$ sont tels que définis précédemment,
réaction conduisant aux composés de formule (IXa) :

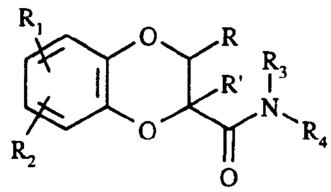

(IXa)

dans laquelle $R_1$, $R_2$, $R_3$, $R_4$, R et R' sont tels que définis précédemment,
cas particulier des composés de formule (I) dans laquelle X représente l'oxygène, qui sont soit :

- transformés par traitement de l'amide correspondant par le réactif de Lawesson, en thioamide de formule (IXb) :

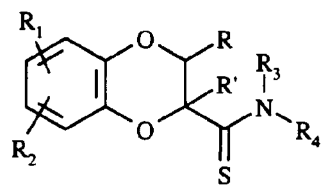

(IXb)

dans laquelle $R_1$, $R_2$, $R_3$, $R_4$, R et R' sont tels que définis précédemment,
cas particulier des composés de formule (I) dans laquelle X représente le soufre,

- soit transformés par réduction de l'amide correspondant grâce à un agent réducteur, tel que l'hydrure double de lithium et d'aluminium, en solvant aprotique anhydre, tel que l'éther éthylique, en composés de formule (IXc) :

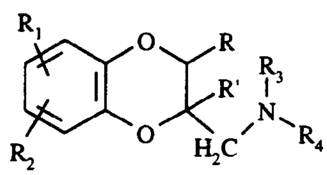

(IXc)

dans laquelle $R_1$, $R_2$, $R_3$, $R_4$, R et R' sont tels que définis précédemment,
cas particulier des composés de formule (I) dans laquelle X représente le groupement $H_2$,

l'ensemble des composés de formules (IXa), (IXb) et (IXc) formant l'ensemble des composés de formule (I) qui sont purifiés et éventuellement séparés en leurs stéréoisomères par une technique classique de séparation et qui sont, si on le désire, transformés en leurs sels d'addition à un acide ou à une base, pharmaceutiquement acceptables.

Les composés de formule (I) dans laquelle $R_3$ et $R_4$ représentent chacun l'hydrogène peuvent également être préparés directement par action d'une solution d'ammoniaque dans l'éthanol sur les composés de formule (V).

Les composés de la présente invention possèdent des propriétés antioxydantes très importantes. Les études pharmacologiques ont notamment montré que les composés de la présente invention sont doués d'activités protectrices remarquables dans le cadre des processus de peroxydation des lipides et en particulier des lipoprotéines. Les activités des composés de l'invention sont notamment très supérieures à celles du probucol, composé commercialisé, connu pour son pouvoir antioxydant et utilisé en thérapeutique.

Les études pharmacologiques ont également permis de mettre en évidence une forte activité protectrice vis-à-vis de cellules placées en conditions oxydantes. Enfin, les produits de l'invention on montré une activité antihypoxique remarquable in vivo chez la souris à des doses très nettement inférieures à celles de la vincamine utilisée comme produit de référence. On peut donc attendre que les composés de la présente invention, qui présentent à la fois des propriétés antioxydantes, protectrices des LDL, inhibitrices de la peroxydation des lipides ainsi qu'une remarquable protection de l'intégrité cellulaire soient particulièrement utiles dans le traitement ou la prévention des affections où interviennent des processus oxydatifs et notamment les désordres ischémiques, les maladies métaboliques, l'athérome, l'artériosclérose, la protection cérébro- et cardiovasculaire ainsi que dans le traitement ou la prévention des dommages dus aux traumatismes chirurgicaux et à la reperfusion d'organes.

Les composés de l'invention ont également montré une activité anticalcique et sont doués d'un pouvoir anti-agrégant plaquettaire.

Les composés de formule (I) peuvent ainsi être utilisés pour l'obtention de médicaments utiles dans le traitement ou la prévention des affections dues ou reliées à des phénomènes de peroxydation, dans le traitement des désordres ischémiques, des maladies métaboliques, de l'athérome, de l'artériosclérose, de la protection cérébro- et cardiovasculaire et dans la prévention et le traitement des dommages dus aux traumatismes chirurgicaux et à la reperfusion d'organes.

La présente invention a également pour objet les compositions pharmaceutiques contenant un dérivé de formule (I), ou un de ses sels d'addition à un acide ou une base pharmaceutiquement acceptables, seul ou en combinaison avec un ou plusieurs excipients inertes et non toxiques. Parmi les compositions pharmaceutiques selon l'invention, on pourra citer plus particulièrement celles qui conviennent à l'administration orale, parentérale, nasale, rectale, perlinguale, oculaire ou pulmonaire et notamment les préparations injectables, les aérosols, les gouttes oculaires ou nasales, les comprimés simples, pelliculés ou dragéifiés, les gélules, les capsules, les crèmes, pommades, gels dermiques,....

La posologie utile varie selon l'âge et le poids du patient, la voie d'administration, la nature de l'affection et des traitements éventuels associés et s'échelonne entre 0,5 mg et 2 grammes par 24 heures. Les exemples suivants illustrent l'invention sans toutefois la limiter en aucune façon. Les matières premières sont disponibles ou préparées à partir de modes opératoires connus.

**PREPARATION 1 : 2,3-DIHYDRO-1,4-BENZODIOXINE-2-CARBOXYLATE D'ETHYLE**

Additionner successivement sous atmosphère d'azote 18,2 g (131 mmol) de carbonate de potassium sec et 13 g (50 mmol) de 2,3-dibromopropionate d'éthyle à une solution de 20 g (181 mmol) de catéchol en solution dans 120 cm³ d'acétone anhydre. L'opération est répétée quatre fois de suite à intervalles de 15 minutes. Le milieu réactionnel est chauffé à reflux pendant 18 heures, puis refroidi et filtré sur célite. Le filtrat est concentré jusqu'à un volume résiduel de 60 cm³ puis hydrolysé avec 80 cm³ d'eau et extrait à l'éther éthylique. Après séchage sur sulfate de magnésium, la phase éthérée est concentrée sous pression réduite et le produit brut purifié par distillation sous pression réduite (Eb=155°C sous 15 mm Hg). On obtient ainsi le 2,3-dihydro-1,4-benzodioxine-2-carboxylate d'éthyle avec un rendement de 80 %.
$n_D^{25}$ = 1,5214

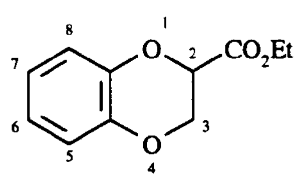

**$^1$H RMN ($CDCl_3$), δ (ppm)** : 1,27 (t, 3H, $CH_2$**$CH_3$**, J = 6,95 Hz) ; 4,25 (q, 2H, **$CH_2$**$CH_3$, J = 6,95 Hz) ; 4,35 (d, 2H, $H_{3a}$, $H_{3b}$, $J_{3,2}$ = 3,79 Hz) ; 4,79 (t, 1H, $H_2$, $J_{2,3}$ = 3,79 Hz) ; 6,86-7,0 (m, 4H, protons aromatiques).

**PREPARATION 2 : 6-ACETYL-1,4-BENZODIOXINE-2-CARBOXYLATE D'ETHYLE**

*Stade 1: 2,3-Dibromo-1,4-benzodioxine-2-carboxylate d'éthyle*

Porter à reflux, pendant 6 heures sous atmosphère d'azote, un mélange de 8 g (38,5 mmol) de 2,3-dihydro-1,4-benzodioxine-2-carboxylate d'éthyle, 15 g (84,5 mmol) de N-bromosuccinimide, une pointe de spatule de 2,2'-azobis-(2-méthylpropionitrile) dans 120 cm³ de tétrachlorure de carbone. Après refroidissement du milieu réactionnel et élimination du succinimide formé, le produit attendu est isolé par mise à sec. On obtient ainsi, le 2,3-dibromo-1,4-benzodioxine-2-carboxylate d'éthyle avec un rendement quantitatif.
Point de fusion : 102° C

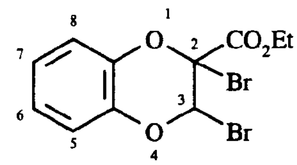

**$^1$H RMN ($CDCl_3$), δ (ppm)** : 1,43 (t, 3H, $CH_2\mathbf{CH_3}$, J = 6,71 Hz) ; 4,41-4,56 (m, 2H, $\mathbf{CH_2}CH_3$) ; 6,94 (s, 1H, $H_3$); 7,1-7,3 (m, $4H_{protons\ aromatiques}$).

*Stade 2: 1,4-Benzodioxine-2 carboxylate d'éthyle*

Agiter pendant 3 heures, à température ambiante et sous atmosphère d'azote, un mélange de 16 g (43,7 mmol) de 2,3-dibromo-1,4-benzodioxine-2-carboxylate d'éthyle et 24 g (160 mmol) d'iodure de sodium dans 80 $cm^3$ d'acetone anhydre. Le milieu réactionnel est ensuite concentré sous pression réduite, hydrolysé avec 80 $cm^3$ d'eau puis extrait à l'éther éthylique. Après traitement avec une solution aqueuse de thiosulfate de sodium puis séchage sur sulfate de magnésium, la phase éthérée est mise à sec sous pression réduite et le produit brut purifié par chromatographie sur colonne de silice (éluant : éther de pétrole/éther éthylique, 40:60). On obtient ainsi le 1,4-benzodioxine-2-carboxylate d'éthyle avec un rendement de 85 %.
Point de fusion : 42°C

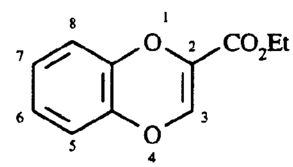

**$^1$H RMN ($CDCl_3$), δ (ppm)** : 1,33 (t, 3H, $CH_2\mathbf{CH_3}$, J = 7,2 Hz) ; 4,26 (q, 2H, $\mathbf{CH_2}CH_3$, J = 7,2 Hz) ; 6,67-6,93 (m, $4H_{protons\ aromatiques}$) ; 6,94 (s, 1H, $H_3$).

*Stade 3: 6-Acétyl-1,4-benzodioxine-2-carboxylate d'éthyle*

Additionner lentement 8,1 g (60,7 mmol) de chlorure d'aluminium à une solution de 2,85 g (36,3 mmol) de chlorure d'acétyle et 5 g (24,7 mmol) de 1,4-benzodioxine-2-carboxylate d'éthyle dans 120 $cm^3$ de sulfure de carbone à 0°C. Agiter 4 heures à température ambiante puis, après hydrolyse acide avec 20 $cm^3$ d'acide chlorhydrique 2N, extraire le milieu réactionnel avec du chlorure de méthylène. Après séchage de la phase chlorométhylénique sur sulfate de magnésium, mise à sec et lavage à l'éthanol, on obtient le 6-acétyl-1,4-benzodioxine-2-carboxylate d'éthyle avec un rendement de 95 %.
Point de fusion : 122°C

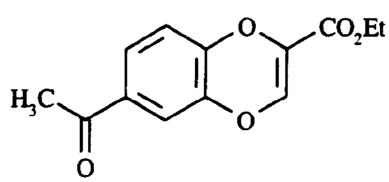

**$^1$H RMN ($CDCl_3$), δ (ppm)** : 1,34 (t, 3H, $CH_2\mathbf{CH_3}$, J = 7,1 Hz) ; 2,51 (s, 3H, $\mathbf{CH_3}CO$) ; 4,29 (q, 2H, $\mathbf{CH_2}CH_3$, J = 7,1 Hz) ; 6,87 (d, 1H, $H_8$, $J_{8,7}$ = 8,69 Hz) ; 7,31 (d, 1H, $H_5$, $J_{5,7}$ = 2,37 Hz) ; 7,53 (dd, 1H, $H_7$, $J_{7,8}$ = 8,69 Hz, $J_{7,5}$ = 2,37 Hz).

**PREPARATION 3 : 6-ETHYL-2,3-DIHYDRO-1,4-BENZODIOXINE-2-CARBOXYLATE D'ETHYLE**

Hydrogéner sous pression d'hydrogène (50 psi) et en présence de 0,750 g de palladium à 5 % sur charbon, une solution de 3 g de 6-acétyl-1,4-benzodioxine-2-carboxylate d'éthyle dans 15 $cm^3$ d'acide acétique. Le produit brut obtenu par mise à sec est purifié par chromatographie sur colonne de silice (éluant : éther de pétrole/éther éthylique, 70:30). On obtient ainsi le 6-éthyl-2,3-dihydro-1,4-benzodioxine-2-carboxylate d'éthyle avec

un rendement de 70 %.

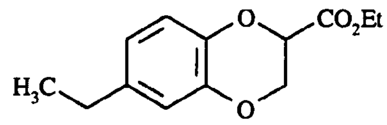

**$^1$H RMN ($CDCl_3$), δ (ppm)** : 6,92 (d, 1H, $H_8$, $J_{8,7}$ = 8,69 Hz) ; 6,74-6,70 (m, 2H, $H_7$, $H_5$, $J_{7,8}$ = 8,69 Hz, $J_{5,7}$ = 1,58 Hz) ; 4,79 (t, 1H, $H_2$, $J_{2,3}$ = 3,95 Hz) ; 4,38-4,36 (m, 2H, $H_{3a}$, $H_{3b}$) ; 4,28 (q, 2H, J = 7,11 Hz, $OCH_2CH_3$) ; 2,56 (q, 2H, J = 7,50 Hz, $CH_3$-$CH_2$-Ar) ; 1,3 (t, 3H, J = 7,11 Hz, $OCH_2CH_3$) ; 1,2 (t, 3H, J = 7,50 Hz, $CH_3CH_2Ar$).

**PREPARATION 4 : 6-ACETYL-2,3-DIHYDRO-1,4-BENZODIOXINE-2-CARBOXYLATE D'ETHYLE**

Méthode A

Additionner 2 g (8,5 mmol) de 6-éthyl-2,3-dihydro-1,4-benzodioxine-2-carboxylate d'éthyle en solution dans 5 cm$^3$ de chlorure de méthylène anhydre à un mélange à 0°C de 0,042 g (0,42 mmol) de trioxyde de chrome et 5,35 g (59,3 mmol) d'hydroperoxyde de tertiobutyle à 70 % dans 30 cm$^3$ de chlorure de méthylène. Agiter 2 heures à 0°C puis 72 heures à température ambiante. Le produit brut, obtenu par mise à sec, est purifié par chromatographie sur colonne de silice (éluant : chlorure de méthylène). On obtient ainsi le 6-acétyl-2,3-dihydro-1,4-benzodioxine-2-carboxylate d'éthyle avec un rendement de 62 %.
Point de fusion : 60°C.

Méthode B

**Préparation du complexe Ag $(Py)_4S_2O_8$**

Ajouter une solution composée de 1,6 g de nitrate d'argent, 32 cm$^3$ d'eau et 14 cm$^3$ de pyridine avec solution aqueuse de 20 g de $K_2S_2O_8$ dans 1350 cm$^3$ d'eau. Le complexe qui précipite est isolé par filtration et séché avant utilisation.
Chauffer à reflux pendant 3 heures un mélange de 1 g de 6-éthyl-2,3-dihydro- 1,4-benzodioxine-2-carboxylate d'éthyle et 7,85 g de complexe Ag $(Py)_4S_2O_8$ dans 20 cm$^3$ d'acétonitrile anhydre. Après refroidissement, élimination de l'insoluble par filtration et concentration sous pression réduite, le produit brut est purifié par chromatographie sur colonne de silice (éluant : éther de pétrole/acétate d'éthyle, 80:20). On obtient ainsi le 6-acétyl-2,3-dihydro-1,4-benzodioxine-2-carboxylate d'éthyle avec un rendement de 78 %.

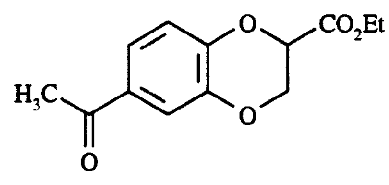

**$^1$H RMN ($CDCl_3$), δ (ppm)** : 7,56 (d, 1H, $H_5$, $J_{5,7}$ = 1,89 Hz) ; 7,52 (dd, 1H, $H_7$, $J_{7,8}$ = 8,05 Hz, $J_{7,5}$ = 1,89 Hz) ; 7,05 (d, 1H, $H_8$, $J_{8,7}$ = 8,05 Hz) ; 4,88 (t, 1H, $H_2$, $J_{2,3}$ = 3,55 Hz) ; 4,41 (q dédoublé, 2H, $H_{3a}$, $H_{3b}$, $J_{3,2}$ = 3,55 Hz, $J_{3a,3b}$ = 11,85 Hz) ; 4,27 (q, 2H, J = 7,35 Hz, $CH_2CH_3$) ; 2,53 (s, 3H, $CH_3CO$); 1,27 (t, 3H, J = 7,35 Hz, $CH_2CH_3$).

**PREPARATION 5 : 7-ACETYL-2,3-DIHYDRO-1,4-BENZODIOXINE-2-CARBOXAMIDE**

*Stade 1: (6 et 7)-Acétyl-2,3-dihydro-1,4-benzodioxine-2-carboxylate d'éthyle*

Additionner sous atmosphère d'azote, 4,5 g (57,7 mmol) de chlorure d'acétyle à une solution de 8 g (38,5 mmol) de 2,3-dihydro-1,4-benzodioxine-2-carboxylate d'éthyle dans 60 cm$^3$ de sulfure de carbone. Refroidir à 0°C puis introduire lentement 12,85 g (96,4 mmol) de chlorure d'aluminium. Agiter 4 heures à température ambiante puis hydrolyser avec 20 cm$^3$ d'une solution glacée d'acide chlorhydrique 2N, et extraire au chlorure de méthylène. Le produit brut obtenu par mise à sec est purifié par chromatographie sur colonne de silice (éluant: éther de pétrole/éther éthylique, 40:60). On obtient ainsi un mélange de 6-acétyl et 7-acétyl-2,3-dihydro-1,4-benzodioxine-2-carboxylate d'éthyle avec un rendement de 80 %.

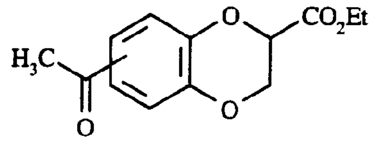

**$^1$H RMN ($CDCl_3$), δ (ppm)** : 7,64-7,5 (m, 4H, $H_{7\text{isomère (2-6)}}$, $H_{5\text{isomère (2-6)}}$, $H_{6\text{isomère (2-7)}}$, $H_{8\text{isomère (2-7)}}$) ; 7,06 (d, 1H, $H_8$, $J_{8,7}$ = 8,29 Hz) ; 6,92 (d, 1H, $H_5$, $J_{5,6}$ = 8,29 Hz) ; 4,92-4,82 (m, 2H, $H_{2\text{isomères (2-6) et (2-7)}}$) ; 4,52-4,38 (m, 4H, 2 x $H_{3a}$,$H_{3b}$) ; 4,28 (q, 4H, 2 x $\mathbf{CH_2}CH_3$, J = 7,1 Hz) ; 2,54 (s, 3H, $\mathbf{CH_3}CO_{\text{isomère (2-7)}}$) ; 2,53 (s, 3H, $\mathbf{CH_3}CO_{\text{isomère (2-6)}}$) ; 1,29 (t, 6H, 2 x $CH_2\mathbf{CH_3}$, J = 7,1 Hz).

*Stade 2: 7-Acétyl-2,3-dihydro-1,4-benzodioxine-2-carboxamide*

Agiter à température ambiante pendant 8 heures une solution de 8 g du mélange de (6 et 7)-acétyl-2,3-dihydro-1,4-benzodioxine-2-carboxylate d'éthyle dans un mélange éthanolammoniac (3:1). Le 7-acétyl-2,3-dihydro-1,4-benzodioxine-2-carboxamide précipite du milieu réactionnel alors que le 6-acétyl-2,3-dihydro-1,4-benzodioxine-2-carboxamide reste en solution. Après isolement par filtration et recristallisation dans l'éthanol à 70 %, le 7-acétyl-2,3-dihydro-1,4-benzodioxine-2-carboxamide est isolé avec un rendement de 50 %.
Point de fusion : 212°C

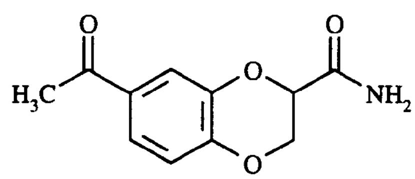

**$^1$H RMN (DMSO), δ (ppm)** : 2,5 (s, 3H, $\mathbf{CH_3}CO$) ; 4,36 (d, 2H, $H_{3a}$, $H_{3b}$, J = 3,95 Hz) ; 4,82 (t, 1H, $H_2$, $J_{2,3}$ = 3,95 Hz) ; 6,97 (d, 1H, $H_5$, $J_{5,6}$ = 8,29 Hz) ; 7,47-7,55 (m, 2H, $H_8$, $H_6$); 7,61 (s large, 2H, $CO\mathbf{NH_2}$)

**PREPARATION 6 : 7-ACETYL-2,3-DIHYDRO-1,4-BENZOMOXINE-2-CARBOXYLATE D'ETHYLE**

Chauffer à reflux pendant 18 heures sous atmosphère d'azote une solution de 3 g (13,5 mmol) de 7-acétyl-2,3-dihydro-1,4-benzodioxine-2-carboxamide en solution dans 100 cm³ d'éthanol saturé en acide chlorhydrique. Après refroidissement, le milieu réactionnel est filtré, concentré, extrait au chlorure de méthylène après neutralisation avec de l'hydrogénocarbonate de sodium. Le produit brut obtenu par mise à sec de la phase organique est purifié par chromatographie sur colonne de silice (éluant : éther de pétrole/éther éthylique, 50:50). On obtient ainsi le 7-acétyl-2,3-dihydro-1,4-benzodioxine-2-carboxylate d'éthyle avec un rendement de 75 %.
Point de fusion : 57°C.

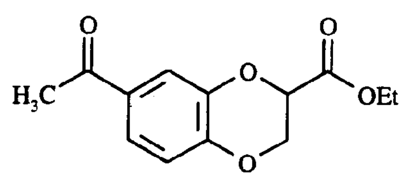

**$^1$H RMN ($CDCl_3$), δ (ppm)** : 1,28 (t, 3H, $CH_2\mathbf{CH_3}$, J = 6,9 Hz) ; 2,53 (s, 3H, $\mathbf{CH_3}CO$); 4,28 (q dédoublé, 2H, $\mathbf{CH_2}CH_3$, J = 6,9 $H_2$); 4,4-4,5 (m, 2H, $H_{3a}$, $H_{3b}$) ; 4,86 (t, 1H, $H_2$, $J_{2,3}$ = 3,55 Hz) ; 6,92 (d, 1H, $H_5$, $J_{5,6}$ = 8,69 Hz) ; 7,52 (dd, 1H, $H_6$, $J_{6,8}$ = 1,97 Hz, $J_{6,5}$ = 8,69 Hz) ; 7,63 (d, 1H, $H_8$, $J_{8,6}$ = 1,97 Hz).

**PREPARATION 7 : 7-ACETOXY-2,3-DIHYDRO-1,4-BENZODIOXINE-2-CARBOXYLATE D'ETHYLE**

Chauffer à 50°C pendant 18 heures sous atmosphère d'azote une solution de 2 g (8 mmol) de 7-acétyl-2,3-dihydro-1,4-benzodioxine-2-carboxylate d'éthyle et 3,05 g (17,7 mmol) d'acide métachloroperbenzoïque

dans 30 $cm^3$ de chlorure de méthylène. Après refroidissement, élimination par filtration de l'acide benzoique formé, et lavages avec des solutions aqueuses d'hydrogénocarbonate de sodium puis de chlorure de sodium, le milieu réactionnel est mis à sec sous pression réduite et le produit brut obtenu est purifié par chromatographie sur colonne de silice (éluant : éther de pétrole/éther éthylique, 70:30). On obtient ainsi le 7-acétoxy-2,3-dihydro-1,4-benzodioxine-2-carboxylate d'éthyle avec un rendement de 75 %.

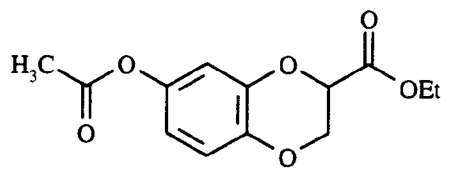

<u>$^1$**H RMN** **($CDCl_3$)**, $\delta$ **(ppm)**</u> : 1,2 (t, 3H, $CH_2$**$CH_3$**) ; 2,26 (s, 3H, **$CH_3$**CO) ; 4,27 (q, 2H, **$CH_2$**$CH_3$, J = 7,1 Hz) ; 4,37 (d, 2H, $H_{3a}$, $H_{3b}$, $J_{2,3}$ = 3,5 Hz) ; 4,83 (t, 1H, $H_2$, J = 1,5 Hz) ; 6,6 (dd, 1H, $H_6$, $J_{6,5}$ = 8,2 Hz, $J_{6,8}$ = 3,16 Hz) ; 6,78 (d, 1H, $H_8$, $J_{8,6}$ = 3,16 Hz) ; 6,84 (d, 1H, $H_5$, $J_{5,6}$ = 8,2 Hz).

**<u>PREPARATION 8</u> : 6-ACETOXY-2,3-DIHYDRO-1,4-BENZODIOXINE-2-CARBOXYLATE D'ETHYLE**

Le 6-acétoxy-2,3-dihydro-1,4-benzodioxine-2-carboxylate d'éthyle est obtenu avec un rendement de 77 % à partir du 6-acétyl-2,3-dihydro-1,4-benzodioxine-2-carboxylate d'éthyle en procédant comme dans la préparation 7.
<u>Point de fusion</u> : 66°C.

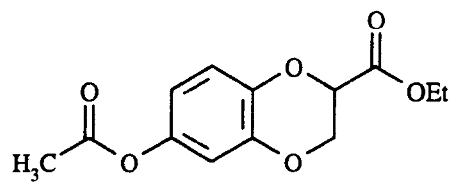

<u>$^1$**H RMN** **($CDCl_3$)**, $\delta$ **(ppm)**</u> : 6,97 (d, 1H, $H_8$, $J_{8,7}$ = 8,53 Hz) ; 6,67-6,58 (m, 2H, $H_7$, $H_5$, $J_{5,7}$ = 2,37 Hz) ; 4,75 (t, 1H, $H_2$, $J_{2,3}$ = 3,55 Hz) ; 4,37 (d, 2H, $H_{3a}$, $H_{3b}$, $J_{3,2}$ = 3,55 Hz) ; 4,27 (q dédoublé, 2H, J = 7,11 Hz, **$CH_2$**$CH_3$) ; 2,22 (s, 3H, **$CH_3$**CO) ; 1,26 (t, 3H, J = 7,11 Hz, **$CH_3$**$CH_2$).

**<u>PREPARATION 9</u> : 7-ACETOXY-1,4-BENZODIOXINE-2-CARBOXYLATE D'ETHYLE**

*<u>Stade 1</u>: 7-Acétoxy-2,3-dibromo-1,4-benzodioxine-2-carboxylate d'éthyle*

En procédant comme dans le stade 1 de la préparation 2 mais en partant de 7-acétoxy-2,3-dihydro-1,4-benzodioxine-2-carboxylate d'éthyle, on obtient le 7-acétoxy-2,3-dibromo- 1,4-benzodioxine-2-carboxylate d'éthyle avec un rendement quasi quantitatif.
<u>Point de fusion</u> : 131°C.

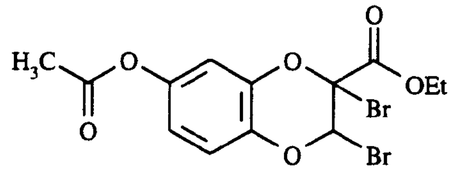

*<u>Stade 2</u>: 7-Acétoxy-1,4-benzodioxine-2-carboxylate d'éthyle*

En procédant comme dans le stade 2 de la préparation 2 mais à partir de 7-acétoxy-2,3-dibromo-1,4-benzodioxine-2-carboxylate d'éthyle, on obtient le 7-acétoxy-1,4-benzodioxine-2-carboxylate d'éthyle avec un rendement de 85 %.
<u>Point de fusion</u> : 66°C.

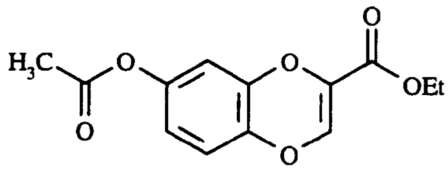

**$^1$H RMN ($CDCl_3$), δ (ppm)** : 1,33 (t, 3H, $CH_2CH_3$) ; 2,26 (s, 3H, $CH_3COO$) ; 4,27 (q, 2H, $CH_2CH_3$, J = 7,11 Hz) ; 6,71-6,58 (m, 3H, $H_8$, $H_6$, $H_5$) ; 6,94 (s, 1H, $H_3$).

**PREPARATION 10 : 6-ACETOXY-1,4-BENZODIOXINE-2-CARBOXYLATE D'ETHYLE**

*Stade 1: 6-Acétyl-2,3-dibromo-1,4-benzodioxine-2-carboxylate d'éthyle*

Additionner goutte à goutte une solution de 0,543 cm³ (10,6 mmol) de brome dans 3 cm³ de tétrachlorure de carbone à 2,5 g (10,1 mmol) de 6-acétyl-1,4-benzodioxine-2-carboxylate d'éthyle partiellement solubilisés dans 35 cm³ de tétrachlorure de carbone à 0°C. Maintenir l'agitation à 0°C pendant 2 heures puis concentrer sous pression réduite et purifier le produit brut obtenu par chromatographie sur colonne de silice (éluant : éther de pétrole/éther éthylique, 60:40). On obtient ainsi le 6-acétyl-2,3-dibromo-1,4-benzodioxine-2-carboxylate d'éthyle avec un rendement de 92 %.

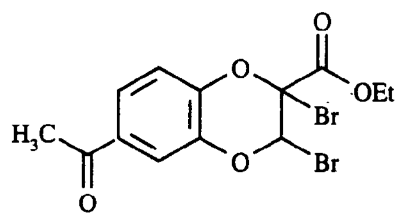

**$^1$H RMN ($CDCl_3$), δ (ppm)** : 7,77 (dd, 1H, $H_7$, $J_{7,8}$ = 8,29 Hz, $J_{7,5}$ = 1,97 Hz) ; 7,70 (d, 1H, $H_5$, $J_{5,7}$ = 1,97 Hz) ; 7,27 (d, 1H, $H_8$, $J_{8,7}$ = 8,29 Hz) ; 6,94 (s, 1H, $H_3$) ; 4,54-4,41 (m, 2H, $CH_2CH_3$) ; 2,59 (s, 3H, $CH_3CO$); 1,44 (t, 3H, J = 7,34 Hz, $CH_3CH_2$).

*Stade 2: 6-Acétoxy-2,3-dibromo-1,4-benzodioxine-2-carboxylate d'éthyle*

En procédant comme pour la préparation 7 mais à partir de 6-acétyl-2,3-dibromo-1,4-benzodioxine-2-carboxylate d'éthyle, on obtient le 6-acétoxy-2,3-dibromo-1,4-benzodioxine-2-carboxylate d'éthyle avec un rendement de 77 %.
Point de fusion : 58°C.

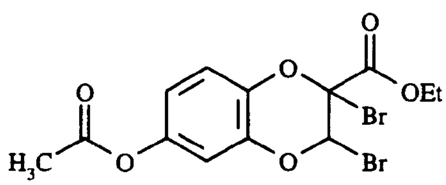

*Stade 3: 6-Acétoxy-1,4-benzodioxine-2-carboxylate d'éthyle*

En procédant comme pour la préparation 9, stade 2, mais à partir de 6-acétoxy-2,3-dibromo-1 ,4-benzodioxine-2-carboxylate d'éthyle on obtient le 6-acétoxy- 1 ,4-benzodioxine-2-carboxylate d'éthyle avec un rendement de 80 %.
Point de fusion : 88°C.

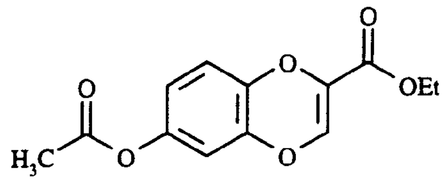

**$^1$H RMN ($CDCl_3$), δ (ppm)** : 6,93 (s, 1H, $H_3$) ; 6,82 (d, 1H, $H_8$, $J_{8,7}$ = 7,7 Hz) ; 6,62 (dd, 1H, $H_7$, $J_{7,8}$ = 7,7 Hz, $J_{7,5}$ = 2,56 Hz) ; 6,51 (d, 1H, $H_5$, $J_{5,7}$ = 2,56 Hz) ; 4,29 (q, 2H, J = 7,2 Hz, $\mathbf{CH_2}CH_3$) ; 2,26 (s, 3H, $\mathbf{CH_3}COO$) ; 1,34 (t, 3H, J = 7,2 Hz, $CH_3\mathbf{CH_2}$).

**PREPARATION 11 : 7-HYDROXY-6-TERTIOBUTYI-1,4-BENZODIOXINE-2-CARBOXYLATE D'ETHYLE**

*Stade 1: 7-Hydroxy-1,4-benzodioxine-2-carboxylate d'éthyle*

Additionner 0,5 cm³ d'une solution molaire d'éthylate de sodium dans l'éthanol à une solution de 1 g (3,78 mmol) de 7-acétoxy-1,4-benzodioxine-2-carboxylate d'éthyle dans 15 cm³ d'éthanol anhydre sous atmosphère d'azote. Agiter 18 heures à température ambiante puis neutraliser avec de la résine Dowex X-8, forme acide, préalablement lavée à l'éthanol. Après filtration et mise à sec sous pression partielle, le produit brut obtenu est purifié par chromatographie sur colonne de silice (éluant : éther de pétrole/éther éthylique, 80:20). On obtient ainsi le 7-hydroxy-1,4-benzodioxine-2-carboxylate d'éthyle avec un rendement de 96 %.
Point de fusion : 160°C.

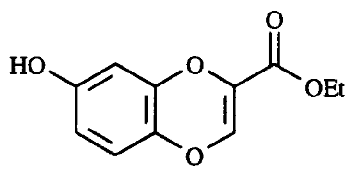

**$^1$H RMN (DMSO), δ (ppm)** : 9,54 (s, 1H, OH) ; 7,22 (s, 1H, $H_3$) ; 6,66 (d, 1H, $H_5$, $J_{5,6}$ = 8,69 Hz) ; 6,29 (dd, 1H, $H_6$, $J_{6,5}$ = 8,69 Hz, $J_{6,8}$ = 2,76 Hz) ; 6,23 (d, 1H, $H_8$, $J_{8,6}$ = 2,76 Hz) ; 4,17 (q, 2H, J = 7,11 Hz, $\mathbf{CH_2}CH_3$) ; 1,22 (t, 3H, J = 7,11 Hz, $\mathbf{CH_3}CH_2$).

*Stade 2: 7-Hydroxy-6-tertiobutyl-1,4-benzodioxine-2-carboxylate d'éthyle*

Ajouter 0,425 cm³ (4,5 mmol) de tertiobutanol à une solution de 0,5 g (2,25 mmol) de 7-hydroxy-1,4-benzodioxine-2-carboxylate d'éthyle dans 3 cm³ d'acide trifluoroacétique. Agiter 24 heures à température ambiante puis, après mise à sec sous pression réduite, le produit brut est purifié par chromatographie sur colonne de silice (éluant : éther de pétrole/éther éthylique, 80:20). On obtient le 7-hydroxy-6-tertiobutyl-1,4-benzodioxine-2-carboxylate d'éthyle avec un rendement de 78 %.
Point de fusion : 222°C.

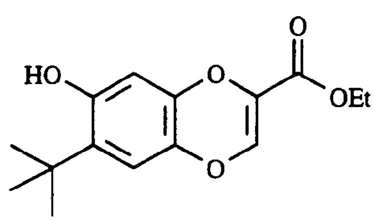

**$^1$H RMN ($CDCl_3$), δ (ppm)** : 6,96 (s, 1H, $H_3$) ; 6,63 (s, 1H, $H_5$) ; 6,31 (s, 1H, $H_8$) ; 5,14 (s, 1H, OH) ; 4,27 (q, 2H, J = 7,3 Hz, $\mathbf{CH_2}CH_3$) ; 1,33 (t, 3H, J = 7,3 Hz, $\mathbf{CH_3}CH_2$).

**PREPARATION 12 : 6-HYDROXY-7-TERTIOBUTYL-1,4-BENZODIOXINE-2-CARBOXYLATE D'ETHYLE**

En procédant comme pour la préparation 11, on obtient le 6-hydroxy-7-tertiobutyl-1,4-benzodioxine-2-carboxylate d'éthyle à partir du 6-acétyl-1,4-benzodioxine-2-carboxylate d'éthyle avec un rendement global de

63 %.
Point de fusion : 125°C.

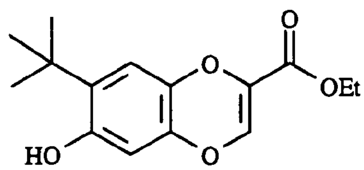

**$^1$H RMN (DMSO), δ (ppm)** : 9,44 (s, 1H, OH) ; 7,16 (s, 1H, $H_3$) ; 6,55 (s, 1H, $H_8$) ; 6,26 (s, 1H, $H_5$) ; 4,18 (q, 2H, J = 7,35 Hz, $\mathbf{CH_2}CH_3$) ; 1,26 (s, 9H, $(\mathbf{CH_3})_3C$) ; 1,22 (t, 3H, J = 7,35 Hz, $\mathbf{CH_3}CH_2$).

**PREPARATION 13 : 7-HYDROXY-6-TERTIOBUTYL-2,3-DIHYDRO-1,4-BENZODIOXINE-2-CARBOXYLATE D'ETHYLE**

En procédant comme pour la préparation 11, on obtient le 7-hydroxy-6-tertiobutyl-2,3-dihydro-1,4-benzodioxine-2-carboxylate d'ethyle à partir du 7-acétoxy-2,3-dihydro- 1,4-benzodioxine-2-carboxylate d'éthyle avec un rendement global de 73 %.
Point de fusion : 128°C.

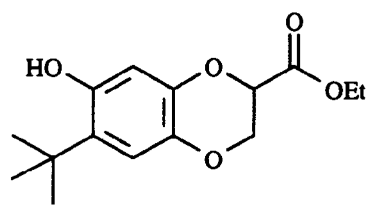

**$^1$H RMN (CDCl$_3$), δ (ppm)** : 6,78 (s, 1H, $H_5$) ; 6,4 (s, 1H, $H_8$) ; 4,78 (t, 1H, $H_2$, J = 3,68 Hz) ; 4,66 (s, 1H, OH) ; 4,33 (d, 2H, $H_{3a}$, $H_{3b}$, J = 3,68 Hz) ; 4,28 (q, 2H, J = 7,35 Hz, $\mathbf{CH_2}CH_3$) ; 1,36 (s, 9H, $(\mathbf{CH_3})_3C$) ; 1,31 (t, 3H, J = 7,35 Hz, $\mathbf{CH_3}CH_2$).

**PREPARATION 14 : 6-HYDROXY-7-TERTIOBUTYL-2,3-DIHYDRO-1,4-BENZODIOXINE-2-CARBOXYLATE D'ETHYLE**

En procédant comme pour la préparation 11, on obtient le 6-hydroxy-7-tertiobutyl-2,3- dihydro- 1,4-benzodioxine-2-carboxylate d'éthyle à partir du 6-acétoxy-2,3-dihydro- 1,4-benzodioxine-2-carboxylate d'éthyle avec un rendement global de 71 %.
Point de fusion : 172°C.

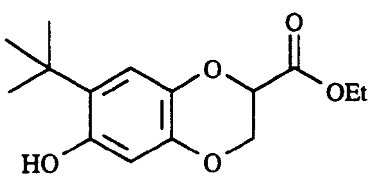

**$^1$H RMN (CDCl$_3$), δ (ppm)** : 6,94 (s, 1H, $H_5$) ; 6,26 (s, 1H, $H_8$) ; 4,73-4,71 (m, 1H, $H_2$) ; 4,52 (s, 1H, OH) ; 4,4-4,23 (m, 4H, $H_{3a}$, $H_{3b}$, $\mathbf{CH_2}CH_3$) ; 1,36 (s, 9H, $(\mathbf{CH_3})_3C$) ; 1,31 (t, 3H, J = 7,3 Hz, $\mathbf{CH_3}CH_2$).

**PREPARATION 15 : ACIDE 7-HYDROXY-2,3-DIHYDRO-1,4-BENZODIOXINE-2-CARBOXYLIQUE**

Ajouter lentement, sous atmosphère d'azote, 75 mmol d'une solution aqueuse d'hydroxyde de sodium à 10 % à une solution de 2 g (7,5 mmol) de 7-acétoxy-2,3-dihydro-1,4-benzodioxine-2-carboxylate d'éthyle dans 20 cm$^3$ de méthanol. Agiter 6 heures à température ambiante puis, après concentration sous pression réduite, acidifier le milieu avec une solution d'acide chlorhydrique 2N. Extraire avec de l'éther éthylique l'acide qui précipite, puis, après séchage et mise à sec, laver l'acide obtenu avec du chlorure de méthylène. On obtient ainsi

l'acide 7-hydroxy-2,3-dihydro-1,4-benzodioxine-2-carboxylique avec un rendement de 85 %.
Point de fusion : 204°C.

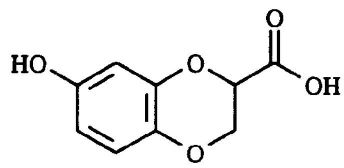

**$^1$H RMN (DMSO), δ (ppm)** : 4,13 (dd, 1H, $H_{3b}$, $J_{3,2}$ = 3,55 Hz, $J_{3b,3a}$ = 11,45 Hz) ; 4,30 (dd, 1H, $H_{3a}$, $J_{3,2}$ = 3,55 Hz, $J_{3a,3b}$ = 11,45 Hz) ; 4,94 (t, 1H, $H_2$, $J_{2,3}$ = 3,55 Hz) ; 6,21 (dd, 1H, $H_6$, $J_{6,5}$ = 8,29 Hz, $J_{6,8}$ = 2,76 Hz) ; 6,3 (d, 1H, $H_8$, $J_{8,6}$ = 2,76 Hz) ; 6,61 (d, 1H, $H_5$, $J_{5,6}$ = 8,29 Hz) ; 9 (s, 1H, OH) ; 13,22 (s large, 1H, COOH).

**PREPARATIONS 16 A 23** :

En procédant comme pour la préparation 15 mais en remplaçant le 7-acétoxy-2,3-dihydro-1,4-benzodioxine-2-carboxylate d'éthyle par :

* le 6-acétoxy-2,3-dihydro-1,4-benzodioxine-2-carboxylate d'éthyle, on obtient l'acide 6-hydroxy-2,3-dihydro-1,4-benzodioxine-2-carboxylique avec un rendement de 90 %.
  Point de fusion : 165°C.

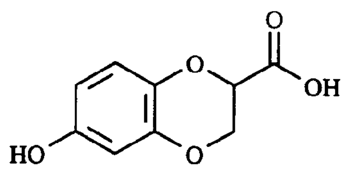

**$^1$H RMN (DMSO), δ (ppm)** : 13 (s large, 1H, COOH) ; 9 (s, 1H, OH), 6,69 (d, 1H, $H_8$, $J_{8,7}$ = 7,58 Hz) ; 6,31-6,19 (m, 2H, $H_5$, $H_7$) ; 4,88 (t, 1H, $H_2$, $J_{2,3}$ = 3,79 Hz) ; 4,36-4,18 (m, 2H, $H_{3a}$, $H_{3b}$).

* le 7-acétoxy-1,4-benzodioxine-2-carboxylate d'éthyle, on obtient l'acide 7-hydroxy-1,4-benzodioxine-2-carboxylique avec un rendement de 88 %.
  Point de fusion : 280°C.

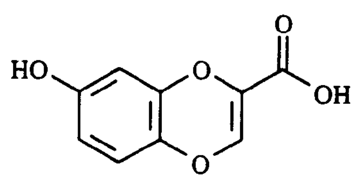

**$^1$H RMN (DMSO), δ (ppm)** : 6,21 (d, 1H, $H_8$, $J_{8,6}$ = 2,84 Hz) ; 6,28 (dd, 1H, $H_6$, $J_{6,5}$ = 8,53 Hz, $J_{6,8}$ = 2,84 Hz) ; 6,65 (d, 1H, $H_5$, $J_{5,6}$ = 8,53 Hz) ; 7,14 (s, 1H, $H_3$) ; 9,5 (s, 1H, OH) ; 13 (s large, 1H, COOH).

* le 6-acétoxy-1,4-benzodioxine-2-carboxylate d'éthyle, on obtient l'acide 6-hydroxy-1,4-benzodioxine-2-carboxylique avec un rendement de 87 %.
  Point de fusion : 260°C.

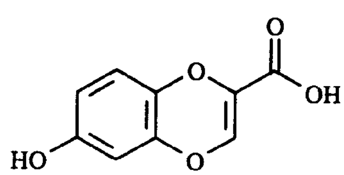

**$^1$H RMN (DMSO), δ (ppm)** : 13 (s large, 1H, COOH) ; 9,44 (s, 1H, HO) ; 7,11 (s, 1H, $H_3$) ; 6,66 (d, 1H, $H_8$, $J_{8,7}$ = 8,29 Hz) ; 6,33 (dd, 1H, $H_7$, $J_{7,8}$ = 8,29 Hz, $J_{7,5}$ = 1,97 Hz) ; 6,24 (d, 1H, $H_5$, $J_{5,7}$ = 1,97 Hz).

* le 7-acétoxy-6-tertiobutyl-1,4-benzodioxine-2-carboxylate d'éthyle, on obtient l'acide 7-hydroxy-6-tertiobutyl- 1 ,4-benzodioxine-2-carboxylique.
Point de fusion : 144°C.

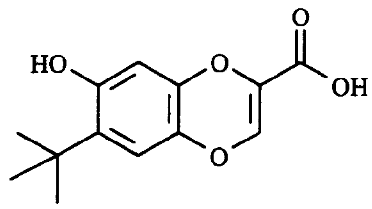

**$^1$H RMN (DMSO), δ (ppm)** : 12,96 (s large, 1H, COOH) ; 9,44 (s, 1H, OH) ; 7,13 (s, 1H, $H_3$) ; 6,53 (s, 1H, $H_5$) ; 6,25 (s, 1H, $H_8$) ; 1,27 (s, 9H, $(CH_3)_3C$).

* le 7-acétoxy-6-tertiobutyl-2,3-dihydro-1,4-benzodioxine-2-carboxylate d'éthyle, on obtient l'acide 7-hydroxy-6-tertiobutyl-2,3-dihydro-1,4-benzodioxine-2-carboxylique.
Point de fusion : 264°C.

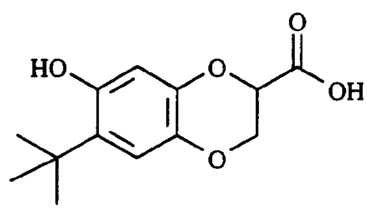

**$^1$H RMN (DMSO), δ (ppm)** : 13 (s, 1H, COOH) ; 8,88 (s, 1H, OH) ; 6,51 (s, 1H, $H_8$) ; 6,34 (s, 1H, $H_5$) ; 4,6-4,4 (m, 1H, $H_2$) ; 4,2-4,0 (m, 2H, $H_{3a}$, $H_{3b}$) ; 1,21 (s, 9H, $(CH_3)_3C$).

* le 6-acétoxy-7-tertiobutyl-1,4-benzodioxine-2-carboxylate d'éthyle, on obtient l'acide 6-hydroxy-7-tertiobutyl-1,4-benzodioxine-2-carboxylique.
Point de fusion : 130°C.

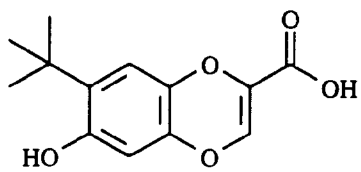

**$^1$H RMN (DMSO), δ (ppm)** : 13 (s large, 1H, COOH) ; 9,4 (s, 1H, OH) ; 7,09 (s, 1H, $H_3$) ; 6,55 (s, 1H, $H_8$) ; 6,26 (s, 1H, $H_5$) ; 1,28 (s, 9H, $(CH_3)_3C$).

* le 6-acétoxy-7-tertiobutyl-2,3-dihydro-1,4-benzodioxine-2-carboxylate d'éthyle, on obtient l'acide 6-hydroxy-7-tertiobutyl-2,3-dihydro-1,4-benzodioxine-2-carboxylique.
Point de fusion : 162°C.

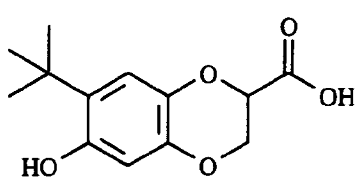

**$^1$H RMN (DMSO), δ (ppm)** : 13,2 (s large, 1H, COOH) ; 8,91 (s, 1H, OH) ; 6,65 (s, 1H, $H_8$) ; 6,31 (s, 1H, $H_5$) ; 4,88 (t, 1H, $H_2$, $J_{2,3}$ = 2,94 Hz) ; 4,33-4,17 (m, 2H, $H_{3a}$, $H_{3b}$) ; 1,27 (s, 9H, $(CH_3)_3C$).

* le 1,4-benzodioxine-2-carboxylate d'éthyle, on obtient l'acide 1,4-benzodioxine-2-carboxylique avec un rendement de 95 %.
Point de fusion : 180°C.

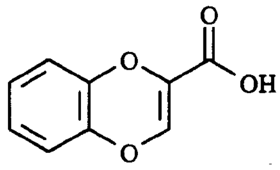

**PREPARATION 24 : ACIDE 6-METHOXY-1,4-BENZODIOXINE-2-CARBOXYLIQUE**

*Stade 1: 6-Hydroxy-1,4-benzodioxine-2-carboxylate de méthyle*

Additionner 3,8 mmol d'une solution 2M de méthylate de sodium à une solution de 0,38 mmol de 6-acétoxy-1,4-benzodioxine-2-carboxylate d'éthyle dans 5 $cm^3$ de méthanol anhydre. Après 4 heures d'agitation à température ambiante et neutralisation avec de la résine Dowex acide, le milieu réactionnel est concentré sous pression réduite et le produit brut obtenu purifié par chromatographie sur colonne de silice (éluant : éther de pétrole/éther éthylique, 80:20). On obtient ainsi le 6-hydroxy-1,4-benzodioxine-2-carboxylate de méthyle avec un rendement de 87 %.

*Stade 2: 6-Méthoxy-1,4-benzodioxine-2-carboxylate de méthyle*

Agiter 15 minutes à température ambiante une solution sous argon de 0,48 mmol de 6-hydroxy-1,4-benzodioxine-2-carboxylate de méthyle dans 5 $cm^3$ de N,N-dimethylformamide en présence de 0,57 mmol d'hydrure de sodium à 60 % en suspension dans l'huile puis additionner 0,72 mmol d'iodure de méthyle. Après 4 heures d'agitation à température ambiante, le milieu réactionnel est concentré sous pression réduite puis extrait avec du chlorure de méthylène. Après mise à sec des phases chlorométhyléniques, le produit brut est purifié par chromatographie sur colonne de silice (éluant : éther de pétrole/acétate d'éthyle, 80:20). On obtient ainsi le 6-méthoxy-1,4-benzodioxine-2-carboxylate de méthyle avec un rendement de 78 %.

*Stade 3: Acide 6-méthoxy-1,4-benzodioxine-2-carboxylique*

Ce composé est obtenu, selon la préparation 15, avec un rendement de 95 % à partir du 6-méthoxy-1,4-benzodioxine-2-carboxylate de méthyle.

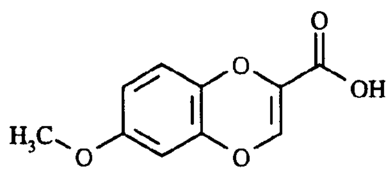

**$^1$H RMN (DMSO), δ (ppm)** : 13,2 (s large, 1H, COOH) ; 7,16 (s, 1H, $H_3$) ; 6,78 (d, 1H, $H_8$, $J_{8,7}$ = 8,9 Hz) ; 6,55-6,45 (m, 2H, $H_7$, $H_5$) ; 3,68 (s, 3H, **$CH_3O$**).

**EXEMPLE 1 : 6-HYDROXY-2-{4-[BIS(4-FLUOROPHENYL)METHYL]PIPERAZIN-1-YL}CARBONYL-1,4-BENZODIOXINE**

Additionner successivement sous atmosphère d'azote 5,5 mmol de 1-[bis(4-fluorophényl)méthyl]pipérazine, 5,5 mmol d'EDCI (1-(3-diméthylaminopropyl)-3-éthylcarbodiimide) et 5,5 mmol d'HOBt (hydroxybenzotriazole) à une solution à 0°C de 5 mmol d'acide 6-hydroxy-1,4-benzodioxine-2-carboxylique dans 25 $cm^3$ de N,N-diméthylformamide. Agiter 2 heures à 0°C puis 12 heures à température ambiante et concentrer le milieu réactionnel sous pression réduite. Extraire l'amide formé avec de l'éther éthylique puis, après mise à sec, purifier le produit brut par chromatographie sur colonne de silice (éluant : éther de pétrole puis gradient croissant d'acétate d'éthyle dans l'éther de pétrole). On obtient ainsi le 6-hydroxy-2-{4-[bis-(4-fluorophényl)méthyl]pipérazin-1-yl}carbonyl-1,4-benzodioxine avec un rendement de 85 %. Le produit obtenu précipite dans 10 ml de pentane, puis est filtré et recristallisé dans le cyclohexane.
Point de fusion : 163°C.

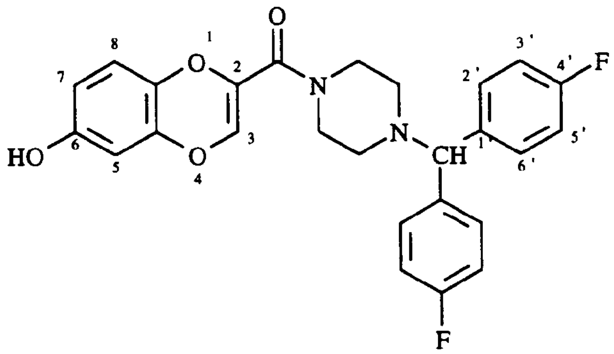

**$^1$H RMN ($CDCl_3$), δ (ppm)** : 7,34 (dd, 4H, 2 x $H_{2'}$, $H_{6'}$, $J_{2',3'}$ = 8,69 Hz, $J_{3',F}$ = 5,53 Hz) ; 6,98 (t, 4H, 2 x $H_{3'}$, $H_{5'}$, $J_{3',2'}$ = $J_{3',F}$ = 8,69 Hz ; 6,57 (s, 1H, $H_3$) ; 6,48 (d, 1H, $H_8$, $J_{8,7}$ = 8,69 Hz) ; 6,29 (dd, 1H, $H_7$, $J_{7,8}$ = 8,69 Hz, $J_{7,5}$ = 3,16 Hz) ; 6,23 (d, 1H, $H_5$, $J_{5,7}$ = 3,16 Hz) ; 5,6 (s, 1H, OH) ; 4,25 (s, 1H, N-CHAr) ; 3,65-3,4 (m, $4H_{pipérazine}$) ; 2,39-2,29 (m, $4H_{pipérazine}$).

**IR (KBr) ν ($cm^{-1}$) :** 3610-2980 (OH), 1665 (C=O), 1210(C-O-C).

Ce composé peut également être obtenu en remplacant le HOBt par la DMAP (4-diméthylaminopyridine).

En procédant de la même façon mais en faisant varier les amines et les acides, on obtient les composés suivants.

**EXEMPLE 2 : 7-HYDROXY-2-{4-[BIS(4-FLUOROPHENYL)METHYL]PIPERAZIN-1-YL}CARBONYL-1,4-BENZODIOXINE**

Ce composé est obtenu avec un rendement de 76 % à partir de la 1-[bis (4-fluorophényl)méthyl]pipérazine et de l'acide 7-hydroxy-1,4-benzodioxine-2-carboxylique.

Point de fusion : 221°C.

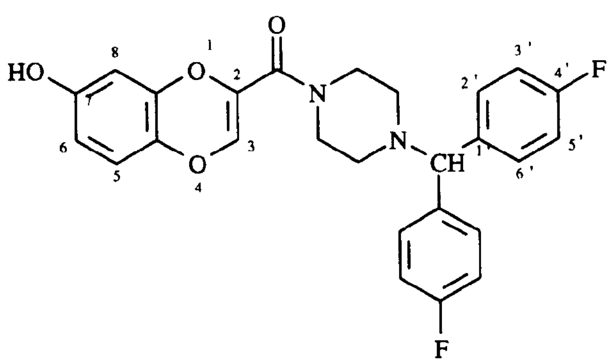

**$^1$H RMN ($CDCl_3$), δ (ppm)** : 7,36 (dd, 4H, 2 x $H_{2'}$, $H_{6'}$, $J_{2',3'}$ = 8,75 Hz, $J_{2',F}$ = 5,63 Hz) ; 7 (t, 4H, 2 x $H_{3'}$, $H_{5'}$, $J_{3',2'}$ = 8,75 Hz, $J_{3',F}$ = 8,75 Hz) ; 6,58-6,52 (m, 2H, $H_3$, $H_5$) ; 6,29 (dd, 1H, $H_6$, $J_{6,5}$ = 8,13 Hz, $J_{6,8}$ = 2,5 Hz) ; 6,23 (s, 1H, $H_8$, $J_{8,6}$ = 2,5 Hz) ; 5,4 (s, 1H, OH) ; 4,27 (s, 1H, N-**CH**-Ar) ; 3,69-3,63 (m, $4H_{pipérazine}$) ; 2,45-2,39 (m, $4H_{pipérazine}$).

**IR (KBr) ν ($cm^{-1}$) :** 3610-3180-2900 (OH), 1650 (C=O), 1210 (=C-O-C).

**EXEMPLE 3 : 6-HYDROXY-2-{4-[BIS(4-FLUOROPHENYL)METHYL]PIPERAZIN-1-YL}CARBONYL-2,3-DIHYDRO-1,4-BENZODIOXINE**

Ce composé est obtenu avec un rendement de 80 % à partir de la 1-[bis-(4-fluorophényl)méthyl]pipérazine et de l'acide 6-hydroxy-2,3-dihydro-1,4-benzodioxine-2-carboxylique.

Point de fusion : 201°C.

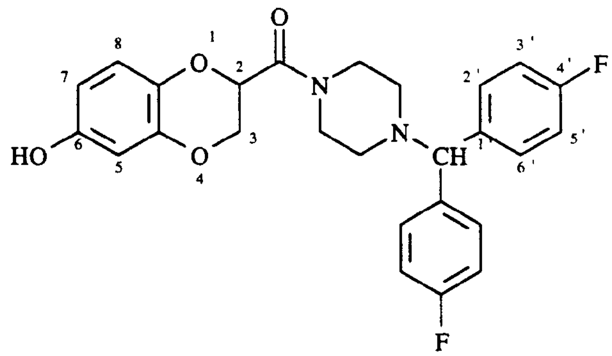

**$^1$H RMN ($CDCl_3$), δ (ppm)** : 7,4-7,3 (m, 4H, 2 x $H_{2'}$, $H_{6'}$) ; 7 (t dédoublé, 4H, 2 x $H_{3'}$, $H_{5'}$, $J_{3',2'}$ = $J_{3',F}$ = 8,69 Hz), 6,71 (d, 1H, $H_8$, $J_{8,7}$ = 8,69 Hz) ; 6,4 (d, 1H, $H_5$, $J_{5,7}$ = 3,16 Hz) ; 6,32 (dd, 1H, $H_7$, $J_{7,5}$ = 3,16 Hz, $J_{7,8}$ = 8,69 Hz) ; 4,92 (s, 1H, OH) ; 4,72 (dd, 1H, $H_2$, $J_1$ = 2,37 Hz, $J_2$ = 7,9 Hz) ; 4,43 (dd, 1H, $H_{3a}$, $J_1$ = 2,37 Hz, $J_2$ = 11,85 Hz) ; 4,29 (dd, 1H, $H_{3b}$, $J_1$ = 5,9 Hz, $J_2$ = 11,85 Hz) ; 4,27 (s, 1H, N-**CH**-Ar) ; 3,82-3,5 (m, $4H_{pipérazine}$) ; 2,5-2,32 m, $4H_{pipérazine}$).

**IR (KBr) ν ($cm^{-1}$)** : 3680-3000 (OH), 1630 (C=O), 1485 (C=C), 1210-1140-1085 (C-O-C)

**EXEMPLE 4 : 7-HYDROXY-2-{4-[BIS(4-FLUOROPHENYL)METHYL]PIPERAZIN-1-YL}CARBONYL-2,3-DIHYDRO-1,4-BENZODIOXINE**

Ce composé est obtenu avec un rendement de 65 % à partir de la 1-[bis(4-fluorophényl)méthyl]pipérazine et de l'acide 7-hydroxy-2,3-dihydro- 1,4-benzodioxine-2-carboxylique.
Point de fusion : 136°C.

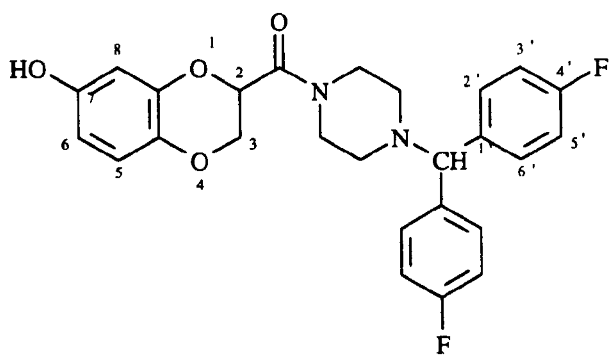

**$^1$H RMN ($CDCl_3$), δ (ppm)** : 7,43-7,31 (m, 4H, 2 x $H_{2'}$, $H_{6'}$) ; 7,04-6,98 (m, 4H, 2 x $H_{3'}$, $H_{5'}$) ; 6,73 (d, 1H, $H_5$, $J_{5,6}$ = 8,2 Hz) ; 6,43 (d, 1H, $H_8$, $J_{8,6}$ = 2,71 Hz) ; 6,34 (dd, 1H, $H_6$, $J_{6,8}$ = 2,71 Hz, $J_{6,5}$ = 8,2 Hz) ; 5,2 (s large, 1H, OH) ; 4,81 (dd, 1H, $H_2$, $J_{2,3b}$ = 2,53 Hz, $J_{2,3a}$ = 3,05 Hz) ; 4,37 (dd, 1H, $H_{3a}$, $J_{3a,2}$ = 3,05 Hz, $J_{3a,3b}$ = 6,32 Hz) ; 4,3-4,16 (m, 2H, **CH**-Ar, $H_{3b}$) ; 3,8-3,5 (m, $4H_{pipérazine}$) ; 2,53-2,29 (m, $4H_{pipérazine}$).

**IR (KBr) ν ($cm^{-1}$)** : 3600-3300-3000 (OH), 1640 (C=O), 1220-1150 (C-O-C).

**EXEMPLE 5 : 7-HYDROXY-6-TERTIOBUTYL-2-{4-[BIS-(4-FLUOROPHENYL)METHYL]PIPERAZIN-1-YL}CARBONYL-2,3-DIHYDRO-1,4-BENZODIOXINE**

Ce composé est obtenu avec un rendement de 72 % à partir de la 1-[bis(4-fluorophényl)méthyl]pipérazine et de l'acide 7-hydroxy-6-tertiobutyl-2,3-dihydro-1,4-benzodioxine-2-carboxylique.
Point de fusion : 165°C.

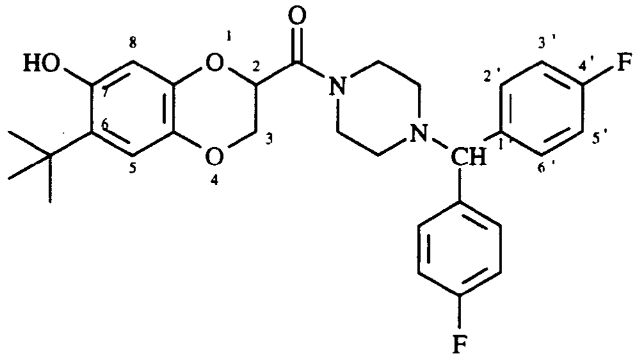

**$^1$H RMN (DMSO), δ (ppm)** : 8,9 (s, 1H, OH) ; 7,45 (dd, 4H, 2 x $H_{2'}$, $H_{6'}$, $J_{2',3'}$ = 8,08 Hz, $J_{2',F}$ = 5,89 Hz) ; 7,14 (t, 4H, 2 x $H_{3'}$, $H_{5'}$, $J_{3',2'}$ = $J_{3',F}$ = 8,08 Hz) ; 6,55 (s, 1H, $H_5$) ; 6,3 (s, 1H, $H_8$) ; 5,04 (dd, 1H, $H_2$, $J_2$ = 2,21 Hz, $J_1$ = 6,62 Hz) ; 4,45 (s, 1H, N-**CH**-Ar) ; 4,22 (dd, 1H, $H_{3a}$, $J_2$ = 2,21 Hz, $J_1$ = 11,76 Hz) ; 4,02 (dd, 1H, $H_{3b}$, $J_1$ = 11,76 Hz, $J_2$ = 6,62 Hz) ; 3,66-3,34 (m, $4H_{pipérazine}$) ; 2,42-2,18 (m, $4H_{pipérazine}$) ; 1,27 (s, 9H, $(\mathbf{CH_3})_3C$).

**IR (KBr) ν (cm$^{-1}$)** : 3640-3060 (OH), 1635 (C=O), 1495 (C=C), 1170-1070 (C-O-C).

**EXEMPLE 6 : 7-HYDROXY-6-TERTIOBUTYL-2-(4-[BIS-(4-FLUOROPHENYL)METHYL]PIPERAZIN-1-YL}CARBONYL-1,4-BENZODIOXINE**

Ce composé est obtenu avec un rendement de 72% à partir de la 1-[bis(4-fluorophényl)méthyl]pipérazine et de l'acide 7-hydroxy-6-tertiobutyl-1,4-benzodioxine-2-carboxylique.

Point de fusion : 184°C.

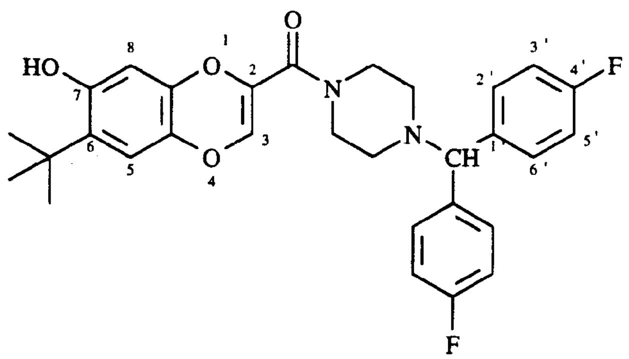

**$^1$H RMN ($CDCl_3$), δ (ppm)** : 7,34 (dd, 4H, 2 x $H_{2'}$, $H_{6'}$, $J_{2',3'}$ = 8,22 Hz, $J_{2',F}$ = 5,05 Hz) ; 7 (t, 4H, 2 x $H_{3'}$, $H_{5'}$, $J_{3',2'}$ = $J_{3',F}$ = 8,22 Hz) ; 6,74 (s large, 1H, OH) ; 6,56 (s, 1H, $H_3$) ; 6,45 (s, 1H, $H_5$) ; 6,24 (s, 1H, $H_8$) ; 4,23 (s, 1H, NCHAr) ; 3,8-3,6 (m, $4H_{pipérazine}$) ; 2,6-2,2 (m, $4H_{pipérazine}$) ; 1,42 (s, 9H, $(CH_3)_3C$).

**IR (KBr) ν (cm$^{-1}$)** : 3680-3210-3000 (OH), 1660 (C=O), 1600-1485 (C=C), 1290-1220 (=C-O-C).

**EXEMPLE 7 : 6-HYDROXY-7-TERTIOBUTYL-2-{4-[BIS(4-FLUOROPHENYL)METHYL]PIPERAZIN-1-YL}CARBONYL-2,3-DIHYDRO-1,4-BENZODIOXINE**

Ce composé est obtenu avec un rendement de 74 % à partir de la 1-[bis(4-fluorophényl)méthyl]pipérazine et de l'acide 6-hydroxy-7-tertiobutyl-2,3-dihydro-1,4-benzodioxine-2-carboxylique.

Point de fusion : 174°C.

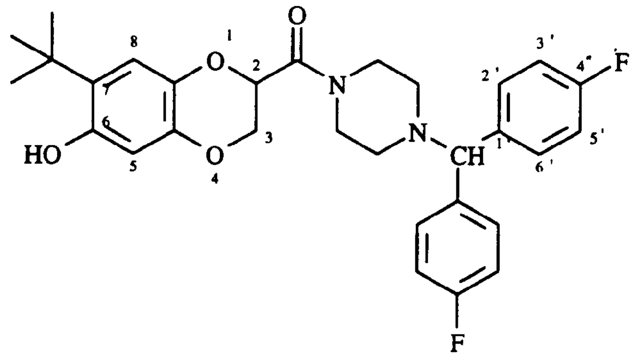

**$^1$H RMN (DMSO), δ (ppm)** : 8,91 (s, 1H, OH) ; 7,45 (dd, 4H, 2 x $H_{6'}$, $H_{2'}$, $J_{2',3'}$ = 8,82 Hz, $J_{2',F}$ = 5,88 Hz) ; 7,14 (t, 4H, $H_{3'}$, $H_{5'}$, $J_{3',2'}$ = $J_{3',F}$ = 8,82 Hz) ; 6,57 (s, 1H, $H_8$) ; 6,27 (s, 1H, $H_5$) ; 4,97 (dd, 1H, $H_2$, $J_1$ = 2,2 Hz, $J_2$ = 7,4 Hz) ; 4,45 (s, 1H, NCHAr) ; 4,26 (dd, 1H, $H_{3a}$, $J_1$ = 2,2 Hz, $J_2$ = 11,8 Hz) ; 4,02 (dd, 1H, $H_{3b}$, $J_2$ = 11,8 Hz, $J_1$ = 7,4 Hz) ; 3,54-3,44 (m, $4H_{pipérazine}$) ; 2,4-2,2 (m, $4H_{pipérazine}$) ; 1,26 (s, 9H, $(CH_3)_3C$).

**IR (KBr) ν ($cm^{-1}$)** : 3640-3000 (OH), 1630 (C=O), 1495 (C=C), 1220-1175 (C-OC).

**EXEMPLE 8 : 6-HYDROXY-7-TERTIOBUTYL-2-{4-[BIS-(4-FLUOROPHENYL)METHYL]PIPERAZIN-1-YL}CARBONYL-1,4-BENZODIOXINE**

Ce composé est obtenu avec un rendement de 83 % à partir de la 1-[bis-(4-fluorophényl)méthyl]pipérazine et de l'acide 6-hydroxy-7-tertiobutyl-1,4-benzodioxine-2-carboxylique.

Point de fusion : 242°C.

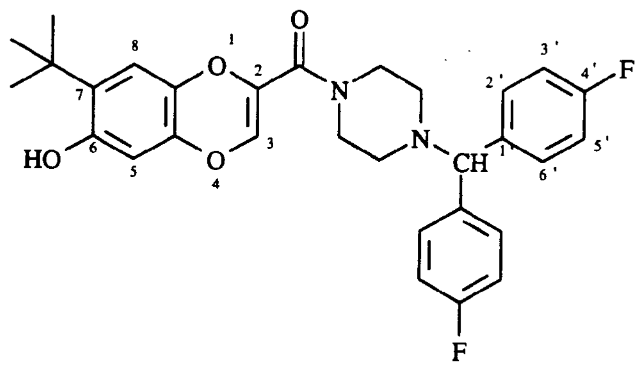

**$^1$H RMN (DMSO), δ (ppm)** : 9,36 (s, 1H, OH) ; 7,43 (dd, 4H, 2 x $H_{2'}$, $H_{6'}$, $J_{2',3'}$ = 8,35 Hz, $J_{2',F}$ = 5,88 Hz) ; 7,12 (t, 4H, 2 x $H_{3'}$, $H_{5'}$, $J_{3',2'}$ = $J_{3',F}$ = 8,35 Hz) ; 6,64 (s, 1H, $H_3$) ; 6,52 (s, 1H, $H_8$) ; 6,24 (s, 1H, $H_5$) ; 4,44 (s, 1H, NCHAr) ; 3,60-3,46 (m, $4H_{pipérazine}$) ; 2,5-2,1 (m, $4H_{pipérazine}$) ; 1,25 (s, 9H $(CH_3)_3C$).

**IR (KBr) ν ($cm^{-1}$)** : 3680-3010 (OH), 1670 (C=O), 1600-1500 (C=C), 1220-1180 (=C-O-C).

**EXEMPLE 9 : 2-{4-[BIS-(4-FLUOROPHENYL)METHYL]PIPERAZIN-1-YL}CARBONYL-1,4-BENZODIOXINE**

Ce composé est obtenu avec un rendement de 78 % à partir de la 1-[bis-(4-fluorophényl) méthyl]pipérazine et de l'acide 1,4-benzodioxine-2-carboxylique.

Point de fusion : 156°C.

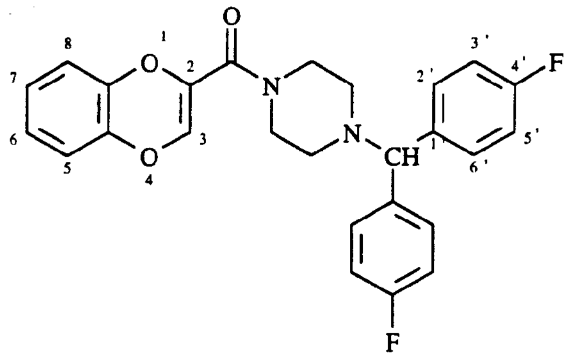

**$^1$H RMN (DMSO), δ (ppm)** : 7,43 (dd, 4H, 2 x $H_{2'}$, $H_{6'}$, $J_{2',3}$ = 8,8 Hz, $J_{2', F}$ = 5,15 Hz) ; 7,12 (t, 4H, 2 x $H_{3'}$, $H_{5'}$, $J_{3',2'}$ = $J_{3', F}$ = 8,8 Hz) ; 6,92-6,78 (m, $4H_{aromatique}$) ; 6,74 (s, 1H, $H_3$) ; 4,43 (s, 1H, N-**CH**-Ar) ; 3,7-3,4 (m, $4H_{pipérazine}$) ; 2,3-2,15 (m, $4H_{pipérazine}$).

**IR (KBr) ν (cm$^{-1}$)** : 3610-3100-3000 (OH), 2980-2780 (CH2, CH3), 1690 (C=C), 1670 (C=O), 1620-1595 (C=C), 1240-1220-1150-1100 (=C-O-C).

**EXEMPLE 10 : 6-HYDROXY-2-ANILINOCARBONYL-1,4-BENZODIOXINE**

Ce composé est obtenu avec un rendement de 72 % à partir de l'aniline et de l'acide 6-hydroxy-1,4-benzodioxine-2-carboxylique.
Point de fusion : 210°C.

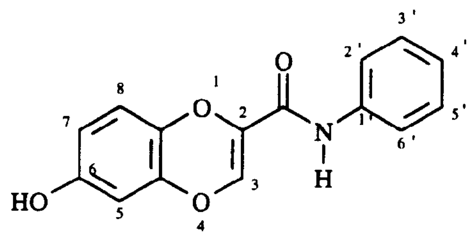

**$^1$H RMN (DMSO), δ (ppm)** : 9,5 (s, 1H, OH) ; 7,68 (d, 2H, 2 x $H_{2'}$, $H_{6'}$, $J_{2',3'}$ = 7,9 Hz) ; 7,32 (t, 2H, $H_{3'}$, $H_{5'}$, J = 7,9 Hz) ; 7,09 (t, 1H, $H_{4'}$, J = 7,9 Hz) ; 7,07 (s, 1H, $H_3$) ; 6,78 (d, 1H, $H_8$, $J_{8,7}$ = 8,69 Hz) ; 6,37 (dd, 1H, $H_7$, $J_{7,8}$ = 8,69 Hz, $J_{7,5}$ = 3,16 Hz) ; 6,27 (d, 1H, $H_5$, $J_{5,7}$ = 3,16 Hz) ; 4,64 (s, 1H, NH).

**IR (KBr) ν (cm$^{-1}$)** : 3610-3000 (OH), 3380 (NH), 1675 (C=O), 1635 (C=C), 1325 (C-O), 1220-1180-1085 (=C-O-C).

**EXEMPLE 11 : 6-HYDROXY-2-ANILINOCARBONYL-2,3-DIHYDRO-1,4-BENZODIOXINE**

Ce composé est obtenu avec un rendement de 75 % à partir de l'aniline et de l'acide 6-hydroxy-2,3-dihydro-1,4-benzodioxine-2-carboxylique.
Point de fusion : 147°C.

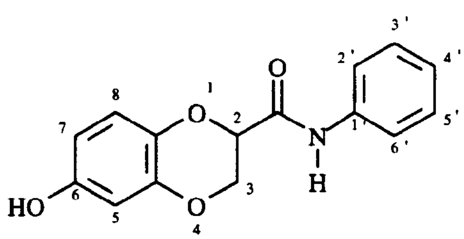

**$^1$H RMN (DMSO), δ (ppm)** : 10 (s, 1H, NH) ; 9,02 (s, 1H, OH) ; 7,62 (d, 2H, $H_{2'}$, $H_{6'}$, J = 7,58) ; 7,30 (t, 2H, $H_{3'}$, $H_{5'}$, J = 7,58 Hz) ; 7,07 (t, 1H, $H_{4'}$, J = 7,1 Hz) ; 6,81 (d, 1H, $H_8$, $J_{8,7}$ = 8,29 Hz) ; 6,29-6,27 (m, 2H, $H_7$, $H_5$) ; 4,81 (dd, 1H, $H_2$, $J_1$ = 2,76 Hz, $J_2$ = 6,32 Hz) ; 4,78 (dd, 1H, $H_{3a}$, $J_1$ = 2,76 Hz, $J_2$ = 11,45 Hz) ; 4,50 (dd, 1H,

$H_{3b}$, $J_1$ = 11,45 Hz, $J_2$ = 6,32 Hz).

**IR (KBr) ν ($cm^{-1}$) :** 3610-3100 (OH), 3370 (NH), 1665 (C=O), 1310-1185-1145 (C-O-C).

**EXEMPLE 12 : 7-HYDROXY-2-ANILINOCARBONYL-2,3-DIHYDRO-1,4-BENZODIOXINE**

Ce composé est obtenu avec un rendement de 65 % à partir de l'aniline et de l'acide 6-hydroxy-2,3-dihydro-1,4-benzodioxine-2-carboxylique.

Point de fusion : 158°C.

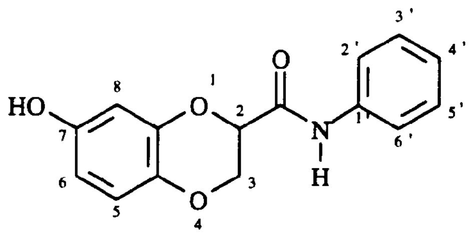

**$^1H$ RMN ($CDCl_3$), δ (ppm)** : 8,33 (s, 1H, NH) ; 7,56 (d, 2H, $H_{2'}$, $H_{6'}$, J = 7,6 Hz) ; 7,35 (t, 2H, $H_{3'}$, $H_{5'}$, J = 7,6 Hz) ; 7,16 (t, 1H, $H_{4'}$, J = 7,6 Hz) ; 6,79 (d, 1H, $H_5$, $J_{5,6}$ = 8,8 Hz) ; 6,57 (d, 1H, $H_8$, $J_{8,6}$ = 3,1 Hz) ; 6,41 (dd, 1H, $H_6$, $J_{6,5}$ = 8,8 Hz, $J_{6,8}$ = 3,1 Hz) ; 5,1 (s, 1H, OH) ; 4,79 (1H, $H_2$, $J_1$ = 7,11 Hz, $J_2$ = 3,16 Hz) ; 4,54 (dd, 1H, $H_{3a}$, $J_1$ = 11,9 Hz, $J_2$ = 3,16 Hz) ; 4,24 (dd, 1H, $H_{3b}$, $J_1$ = 11,9 Hz, $J_2$ = 7,11 Hz).

**IR (KBr) ν ($cm^{-1}$) :** 3500-3000 (OH, NH), 3310 ($NH_{associée}$), 1635 (C=O), 1100-1030 (C-O-C).

**EXEMPLE 13 : 7-HYDROXY-2-ANILINOCARBONYL-1,4-BENZODIOXINE**

Ce composé est obtenu avec un rendement de 63 % à partir de l'aniline et de l'acide 7-hydroxy-1,4-benzodioxine-2-carboxylique.

Point de fusion : 206°C.

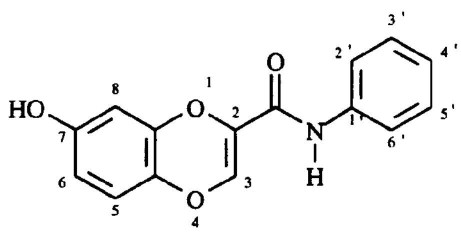

**$^1H$ RMN ($CDCl_3$), δ (ppm)** : 7,83 (s, 1H, NH) ; 7,59 (d, 2H, $H_{2'}$, $H_{6'}$, J = 7,9 Hz) ; 7,36 (t, 2H, $H_{3'}$, $H_{5'}$, J = 7,9 Hz) ; 7,16 (t, 1H, $H_{4'}$) ; 6,64 (d, 1H, $H_5$, $J_{5,6}$ = 8,3 Hz) ; 6,38 (d, 1H, $H_8$, $J_{8,6}$ = 2,8 Hz) ; 6,36 (dd, 1H, $H_6$, $J_{6,8}$ = 2,8 Hz, $J_{6,5}$ = 2,3 Hz) ; 4,95 (s, 1H, OH).

**IR (KBr) ν ($cm^{-1}$)** : 3500-2980 (NH, OH), 3310 ($NH_{associée}$), 1640 (C=O), 1220-1070 (C-O-C).

**EXEMPLE 14 : 6-HYDROXY-2-(N-BUTYLANILINO)CARBONYL-1,4-BENZODIOXINE**

Ce composé est obtenu avec un rendement de 91 % à partir de la N-butylaniline et de l'acide 6-hydroxy-1,4-benzodioxine-2-carboxylique.

Point de fusion : 124°C.

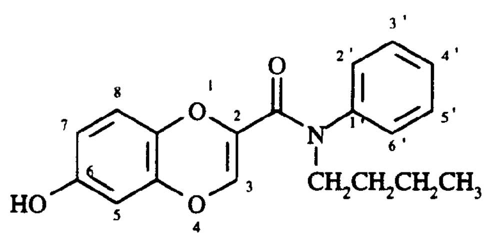

$^1H$ **RMN ($CDCl_3$), δ (ppm)** : 7,4-7,18 (m, $5H_{aromatique}$, N-Ar) ; 6,62 (s, 1H, $H_3$) ; 6,14 (d, 1H, $H_5$, $J_{5,7}$ = 2,9 Hz) ; 6 (dd, 1H, $H_7$, $J_{7,8}$ = 8,3 Hz, $J_{7,5}$ = 2,9 Hz) ; 5,71 (d, 1H, $H_8$, $J_{8,7}$ = 8,3 Hz) ; 5,14 (s, 1H, OH) ; 3,9-3,7 (m, 2H, $N\mathbf{CH_2}CH_2$) ; 1,56-1,47 (m, 2H, $CH_2\mathbf{CH_2}CH_3$) ; 1,5-1,2 (m, 2H, $\mathbf{CH_2}CH_3$) ; 0,88 (t, 3H, J = 7,3 Hz, $CH_2\mathbf{CH_3}$).

**IR (KBr) ν ($cm^{-1}$)** : 3610-2980 (OH), 3310 (NH), 1660 (C=O), 1595-1575-1490 (C=C), 1290-1175-1130-1090 (=C-O-C).

**EXEMPLE 15 : 7-HYDROXY-2-(N-BUTYLANILINO)CARBONYL-1,4-BENZODIOXINE**

Ce composé est obtenu avec un rendement de 78 % à partir de la N-butylaniline et de l'acide 7-hydroxy-1,4-benzodioxine-2-carboxylique.

Point de fusion : 156°C.

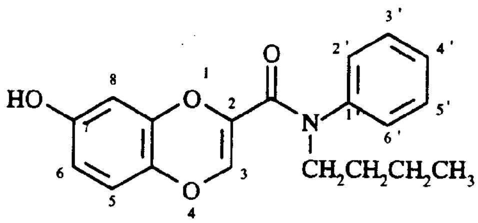

$^1H$ **RMN ($CDCl_3$), δ (ppm)** : 7,43-7,17 (m, $5H_{aromatique}$) ; 6,6 (d, 1H, $H_8$, $J_{8,6}$ = 2,9 Hz) ; 6,44 (d, 1H, $H_5$, $J_{5,6}$ = 8,7 Hz) ; 6,42 (s, 1H, $H_3$) ; 6,20 (dd, 1H, $H_6$, $J_{6,8}$ =2,9 Hz, $J_{6,5}$ = 8,7 Hz) ; 5,56 (s, 1H, OH) ; 3,76 (t, 2H, $N\mathbf{CH_2}CH_2$, J = 7,8 Hz) ; 1,7-1,48 (m, 2H, $NCH_2\mathbf{CH_2}CH_2$) ; 1,43-1,26 (m, 2H, $NCH_2CH_2\mathbf{CH_2}CH_3$) ; 0,89 (t, 3H, J = 7,31 Hz, $CH_2\mathbf{CH_3}$).

**IR (KBr) ν ($cm^{-1}$)** : 3610-2980 (OH), 3210 (NH), 1660 (C=O), 1590-1570-1485 (C=C), 1220-1155-1070(=C-O-C).

**EXEMPLE 16 : 7-HYDROXY-2-[4-(2,3,4-TRIMETHOXYBENZYL)PIPERAZIN-1-YL]CARBONYL-1,4-BENZODIOXINE**

Ce composé est obtenu avec un rendement de 73 % à partir de la 1-(2,3,4-triméthoxybenzyl)pipérazine et de l'acide 7-hydroxy-1,4-benzodioxine-2-carboxylique.

Point de fusion : 93°C.

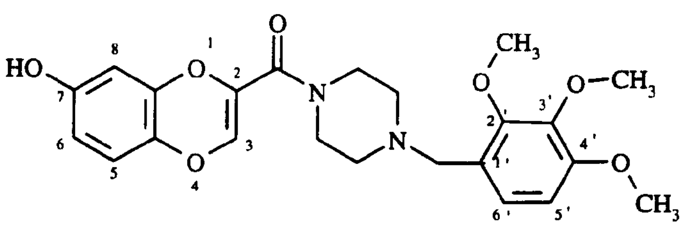

$^1H$ **RMN ($CDCl_3$), δ (ppm)** : 2,55-2,46 (m, $4H_{pipérazine}$) ; 3,51 (s, 2H, N-$\mathbf{CH_2}$-Ar) ; 3,68-3,6 (m, $4H_{pipérazine}$) ; 3,87-3,89 (3s, 9H, 3 x $\mathbf{CH_3}O$) ; 6,25 (d, 1H, $H_8$, $J_{8,6}$ = 2,08 Hz) ; 6,31 (dd, 1H, $H_6$, $J_{6,8}$ = 2,08 Hz, $J_{6,5}$ = 8,33 Hz) ; 6,53 (d, 1H, $H_5$, $J_{5,6}$ = 8,33 Hz) ; 6,54 (s, 1H, $H_3$) ; 6,63 (d, 1H, $J_{1',2'}$ = 8,3 Hz) ; 6,97 (d, 1H, $J_{2',1'}$ = 8,3 Hz).

**IR (KBr) ν ($cm^{-1}$)** : 3610-3010 (OH), 2980 ($ArOCH_3$), 1640 (C=O), 1250-1080 (=C-O-C).

**EXEMPLE 17 : 7-HYDROXY-2-[4-(2,3,4-TRIMETHOXYBENZYL)PIPERAZIN-1-YL]CARBONYL-2,3-DIHYDRO-1,4-BENZODIOXINE**

Ce composé est obtenu avec un rendement de 65 % à partir de la 1-(2,3,4-triméthoxybenzyl)pipérazine et de l'acide 7-hydroxy-2,3-dihydro- 1,4-benzodioxine-2-carboxylique.

Point de fusion : 88°C.

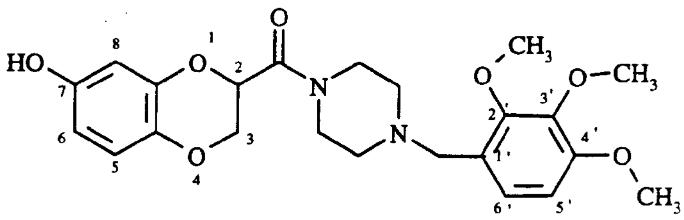

**$^1$H RMN ($CDCl_3$), δ (ppm)** : δ = 2,42-2,52 (m, $4H_{pipérazine}$; 3,54 (s, 2H, N-$CH_2$-Ar) ; 3,52-3,78 (m, $4H_{pipérazine}$) ; 3,69-3,87-3,89 (3s, 9H, $CH_3O$) ; 4,21 (dd, 1H, $H_{3b}$, $J_2$ = 11,44 Hz, $J_1$ = 8,32 Hz) ; 4,4 (dd, 1H, $H_{3a}$, $J_1$ = 2,08 Hz, $J_{3',3}$ = 11,4 Hz) ; 4,82 (dd, 1H, $H_2$, $J_2$ = 8,32 Hz, $J_1$ = 2,08 Hz) ; 6,33 (dd, 1H, $H_6$, $J_{6,8}$ = 2,5 Hz, $J_{6,5}$ = 8,12 Hz) ; 6,44 (d, 1H, $H_8$, $J_{8,6}$ = 2,5 Hz) ; 6,64 (d, 1H, $H_5$, $J_{5,6}$ = 8,12 Hz) ; 6,74 (d, 1H, $H_{5'}$, J = 8,5 Hz) ; 6,97 (d, 1H, $H_{6'}$, J = 8,5 Hz).

**IR (KBr) ν ($cm^{-1}$)** : 3610-3000 (OH), 1660 (C=O), 1590 (C=C), 1150-1080 (C-O-C).

**EXEMPLES 18 A 48**

En procédant de la même façon, on obtient :

***EXEMPLE 18*** : **6-Hydroxy-7-tertiobutyl-2-pipépiridinocarbonyl-1,4-benzodioxine**

***EXEMPLE 19*** : **6-Hydroxy-7-tertiobutyl-2-hexaméthylèneiminocarbonyl-1,4-benzodioxine**

***EXEMPLE 20*** : **6-Hydroxy-7-tertiobutyl-2-pyrrolidinocarbonyl-1,4-benzodioxine**

***EXEMPLE 21*** : **6-Hydroxy-2-(4-phénylpipéridinocarbonyl)-1,4-benzodioxine**

***EXEMPLE 22*** : **6-Hydroxy-2-(4-benzylpipéridinocarbonyl)-1,4-benzodioxine**

***EXEMPLE 23*** : **6-Hydroxy-2-(4-méthylpipéridinocarbonyl)-1,4-benzodioxine**

***EXEMPLE 24*** : **6-Hydroxy-2-[4-(4-fluorobenzoyl)pipéridinocarbonyl]-1,4-benzo-dioxine**

***EXEMPLE 25*** : **6-Hydroxy-2-{4-[bis-(4-fluorophényl)méthylène]pipéridino}carbonyl -1,4-benzodioxine**

***EXEMPLE 26*** : **2-{4-[bis-(4-Fluorophényl)méthyl]pipéridinocarbonyl}-1,4-benzodioxine**

***EXEMPLE 27*** : **6-Hydroxy 2-[N-(n-but-1-yl)aminocarbonyl]-1,4-benzodioxine**

***EXEMPLE 28*** : **6-Hydroxy-2-(N,N-dipropylaminocarbonyl)-1,4-benzodioxine**

***EXEMPLE 29*** : **6-Hydroxy-7-tertiobutyl-2-(N-méthylanilinocarbonyl)-1,4-benzodioxine**

***EXEMPLE 30*** : **6-Hydroxy-7-tertiobutyl-2-(N-butylanilinocarbonyl)-1,4-benzodioxine**

***EXEMPLE 31*** : **6-Hydroxy-2-(3-méthoxyanilino)carbonyl-1,4-benzodioxine**

***EXEMPLE 32*** : **6-Ethyl-2-anilinocarbonyl-1,4-benzodioxine**

***EXEMPLE 33*** : **6-Hydroxy-2-(3,4,5-triméthoxyanilino)carbonyl-1,4-benzodioxine**

***EXEMPLE 34*** : **6-Hydroxy-7-tertiobutyl-2-(N-méthyl-N-benzyl)aminocarbonyl-2,3-dihydro-1,4-benzo-dioxine**

***EXEMPLE 35*** : **6-Hydroxy-2-(quinol-3-yl)aminocarbonyl-1,4-benzodioxine**

***EXEMPLE 36*** : **6-Hydroxy-2-(pyridin-2-ylamino)carbonyl-1,4-benzodioxine**

***EXEMPLE 37*** **: 6-Hydroxy-2-[(2,4-diméthylpyridin-6-yl)aminocarbonyl]-1,4-benzodioxine**

***EXEMPLE 38*** **: 6-Hydroxy-7-tertiobutyl-2-[(2,4-diméthylpyridin-6-yl)aminocarbonyl]-1,4-benzodioxine**

***EXEMPLE 39*** **: 6-Hydroxy-2-(4-propylpipérazin-1-yl)carbonyl-2,3-dihydro-1,4-benzodioxine**

***EXEMPLE 40*** **: 7-Hydroxy-2-[4-(4-fluorophényl)pipérazin-1-yl]carbonyl-2,3-dihydro-1,4-benzodioxine**

***EXEMPLE 41*** **: 6-Hydroxy-2-[4-(pyrimid-2-yl)pipérazin-1-yl]carbonyl-1,4-benzodioxine**

***EXEMPLE 42*** **: 6-Hydroxy-2-(4-benzylpipérazin-1-yl)carbonyl-1,4-benzodioxine**

***EXEMPLE 43*** **: 6-Hydroxy-2-[4-(4-fluorobenzoyl)pipérazin-1-yl]carbonyl-1,4-benzodioxine**

***EXEMPLE 44*** **: 6-Hydroxy-2-(4-phénéthylpipérazin-1-yl)carbonyl-1,4-benzodioxine**

***EXEMPLE 45*** **: 2-(4-[bis-(4-Fluorophényl)méthyl]pipérazin-1-yl)carbonyl-2,3-dihydro-1,4-benzodioxine**

***EXEMPLE 46*** **: 6-Hydroxy-2-{4-[bis-(phényl)méthyl]pipérazin-1-ylcarbonyl}-1,4-benzodioxine**

***EXEMPLE 47*** **: 6-Acétyl-2-{4-[bis-(4-fluorophényl)méthyl]pipérazin-1-yl}carbonyl-1,4-benzodioxine**

***EXEMPLE 48*** **: 7-Acétyl-2-{4-[bis-(4-fluorophényl)méthyl]pipérazin-1-yl}carbonyl-1,4-benzodioxine**

**EXEMPLES 49 ET 50**

En procédant comme pour la préparation 5, on obtient :

***EXEMPLE 49*** **: 6-Acétyl-1,4-benzodioxine-2-carboxamide**

Composé préparé à partir du 6-acétyl-1,4-benzodioxine-2-carboxylate d'éthyle.

***EXEMPLE 50*** **: 6-Acétyl-2,3-dihydro-1,4-benzodioxine-2-carboxamide**

Composé préparé à partir du 6-acétyl-2,3-dihydro-1,4-benzodioxine-2-carboxylate d'éthyle

**EXEMPLE 51 : 6-METHOXY-2-{4-[BIS-(4-FLUOROPHENYL)METHYL] PIPERAZIN-1-YL}CARBONYL-1,4-BENZODIOXINE**

Additionner 0,98 mmol d'hydrure de sodium à 60% en suspension dans de l'huile à une solution de 0,82 mmol de 6-hydroxy-2-{4-[bis-(4-fluorophényl)méthyl] pipérazin-1- yl}carbonyl-1,4-benzodioxine dans 10 $cm^3$ de N,N-diméthylformamide sous atmosphère d'azote. Agiter 15 minutes à température ambiante puis ajouter goutte à goutte 0,98 mmol d'iodure de méthyle. Agiter 2 heures à température ambiante puis mettre à sec sous pression réduite et purifier le produit brut obtenu par chromatographie sur colonne de silice (éluant : éther de pétrole/acétate d'éthyle, 50:50). On obtient ainsi la 6-méthoxy-2-{4-[bis-(4-fluorophényl)méthyl]piperazin-1-yl} carbonyl-1,4-benzodioxine avec un rendement de 83 %.
Point de fusion : 65°C.

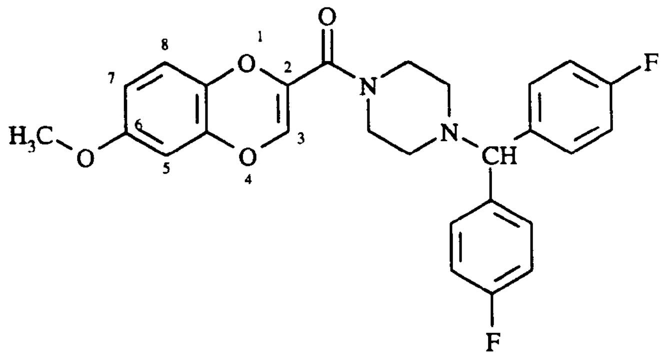

**$^1$H RMN ($CDCl_3$), δ (ppm)** : 7,34 (dd, 4H, 2 x $H_{2'}$, $H_{6'}$, $J_{2',3'}$ = 8,63 Hz, $J_{2',F}$ = 5,4 Hz) ; 6,98 (t, 4H, 2 x $H_{3'}$, $H_{5'}$, $J_{3',2'}$ = $J_{3',F}$ = 8,63 Hz) ; 6,56 (d, 1H, $H_8$, $J_{8,7}$ = 8,8 Hz) ; 6,56 (s, 1H, $H_3$) ; 6,36 (dd, 1H, $H_7$, $J_{7,8}$ = 8,8 Hz, $J_{7,5}$ = 2,9 Hz) ; 6,28 (d, 1H, $H_5$, $J_{5,7}$ = 2,9 Hz) ; 4,25 (s, 1H, NCHAr) ; 3,70 (s, 3H, $CH_3O$) ; 3,72-3,66 (m, $4H_{pipérazine}$) ; 2,5-2,3 (m,$4H_{pipérazine}$).

**IR (KBr) ν ($cm^{-1}$) :** 1660 (C=O), 1605 (C=C), 1575-1485 ($C=C_{Ar}$), 1215-1190-1125-1080 (=C-O-C)

Ce composé peut également être obtenu en faisant réagir la 1-[bis-(4-fluorophényl)méthyl]pipérazine avec l'acide 6-méthoxy-1,4-benzodioxine-2-carboxylique dans les conditions de l'exemple 1.

**EXEMPLE 52 : 7-METHOXY-2-{4-[BIS-(4-FLUOROPHENYL)METHYL]PIPERAZIN-1-YL}CARBONYL-1,4-BENZODIOXINE**

Ce composé est obtenu avec un rendement de 78 % à partir de la 7-hydroxy 2-{4-[bis-(4-fluorophényl)méthyl]pipérazin-1-yl}carbonyl-1,4-benzodioxine selon les conditions de l'exemple 51.

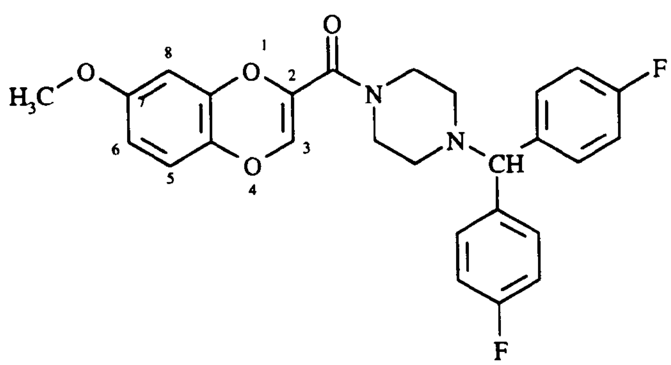

**$^1$H RMN ($CDCl_3$), δ (ppm)** : 7,34 (dd, 4H, 2 x $H_{2'}$, $H_{6'}$, $J_{2',3'}$ = 8,29 Hz, $J_{2',F}$ = 5,53 Hz) ; 6,98 (t, 4H, 2 x $H_{3'}$, $H_{5'}$, $J_{3',2'}$ = $J_{3',F}$ = 8,29 Hz) ; 6,62 (d, 1H, $H_5$, $J_{5,6}$ = 9,1 Hz) ; 6,6 (s, 1H, $H_3$) ; 6,36 (dd, 1H, $H_6$, $J_{6,5}$ = 9,1 Hz, $J_{6,8}$ = 2,96 Hz) ; 6,23 (d, 1H, $H_8$, $J_{8,6}$ = 2,96 Hz) ; 4,27 (s, 1H, NCHAr) ; 3,71 (s, 3H, $CH_3O$) ; 3,7-3,66 (m, $4H_{pipérazine}$) ; 2,45-2,4 (m, $4H_{pipérazine}$).

**EXEMPLE 53 : 6-NICOTINOYLOXY-2-{4-[BIS-(4-FLUOROPHENYL)METHYL]PIPERAZIN-1-YL}CARBONYL-1,4-BENZODIOXINE**

Porter à reflux 8 heures sous atmosphère d'azote une solution de 1,37 mmol de 6-hydroxy-2-{4-[bis-(4-fluorophényl)méthyl]pipérazin-1-y}carbonyl-1,4-benzodioxine, 4,5 mmol de chlorure de nicotinoyle, 3,42 mmol de pyridine anhydre dans 25 $cm^3$ de dichloroéthane. Après refroidissement, le milieu réactionnel est lavé avec une solution aqueuse saturée d'hydrogénocarbonate de sodium, séché sur sulfate de magnésium puis concentré sous pression réduite. Le produit brut est ensuite purifié par chromatographie sur colonne de silice (éluant : éther de pétrole/acétate d'éthyle, 70:30). On obtient ainsi la 6-nicotinoyloxy 2-{4-[bis-(4-fluorophényl)méthyl]pipérazin-1-yl}carbonyl-1,4-benzodioxine avec un rendement de 95 %.

Point de fusion : 142°C.

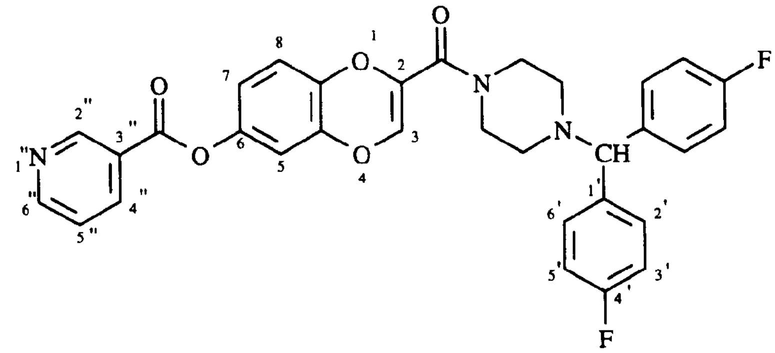

**$^1$H RMN ($CDCl_3$), δ (ppm)** : 9,32 (d, $1H_{pyridine}$, $H_{2''}$, $J_{2'',6''}$ = 1,9 Hz) ; 8,94 (dd, $1H_{pyridine}$, $H_{6''}$, $J_{6'',2''}$ = 1,9 Hz, $J_{6'',5''}$ = 5,1 Hz) ; 8,39 (ddd, $1H_{pyridine}$, $H_{4''}$, $J_{4'',5''}$ = 8 Hz, $J_{4'',2''}$ = 2 Hz) ; 7,45 (dd, $1H_{pyridine}$, $H_{5''}$, $J_{5'',6''}$ = 5,1 Hz, $J_{5'',4''}$ = 8 Hz) ; 7,35 (dd, 4H, 2 x $H_{2'}$, $H_{6'}$, $J_{2',3'}$ = 8,5 Hz, $J_{2',F}$ = 5,35 Hz) ; 6,99 (t, 4H, 2 x $H_{3'}$, $H_{5'}$, $J_{3',2'}$ = $J_{3',F}$ = 8,5 Hz) ; 6,75-6,62 (m, 3H, $H_8$, $H_7$, $H_5$) ; 6,58 (s, 1H, $H_3$) ; 4,26 (s, 1H, N**CH**Ar) ; 3,7-3,5 (m, $4H_{pipérazine}$) ; 2,6-2,3 (m, $4H_{pipérazine}$).

**IR (KBr) ν ($cm^{-1}$)** : 2950-2740 ($CH_3$, $CH_2$), 1740 (C = O), 1675 (C = O), 1615 (C = C), 1265-1165 (=C-O-C).

**EXEMPLE 54 : 7-NICOTINOYLOXY-2-{4-[BIS-(4-FLUOROPHENYL)METHYL]PIPERAZIN-1-YL}CARBONYL-1,4-BENZODIOXINE**

Ce composé est obtenu avec un rendement de 78 % à partir de la 7-hydroxy-2-{4-[bis-(4-fluorophényl)méthyl]pipérazin-1-yl}carbonyl-1,4-benzodioxine et du chlorure de nicotinoyle en procédant comme pour l'exemple 53.

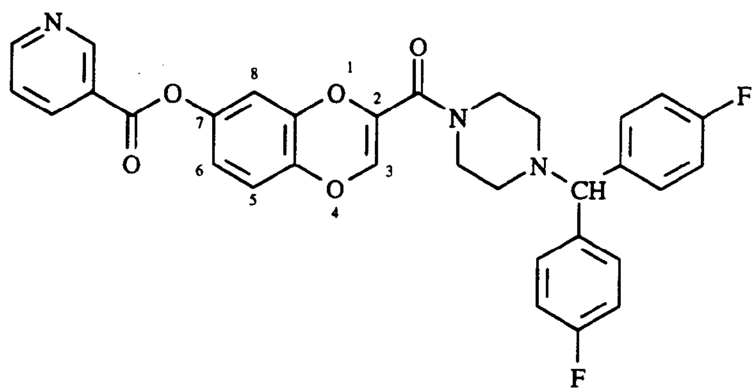

**$^1$H RMN ($CDCl_3$δ (ppm)**: 9,33 (d, $1H_{pyridine}$, $H_{2''}$, $J_{2'',6''}$ = 2,19 Hz) ; 8,85 (dd, $1H_{pyridine}$, $H_{6''}$, $J_{6'',5''}$ = 4,9 Hz, $J_{6'',4''}$ = 1,8 Hz) ; 8,38 (ddd, $1H_{pyridine}$, $H_{4''}$, $J_{4'',6''}$ = 1,8 Hz, $J_{4'',5''}$ = 7,9 Hz) ; 7,45 (dd, $1H_{pyridine}$, $H_{5''}$, $J_{5'',4''}$ = 7,9 Hz, $J_{5'',6''}$ = 4,9 Hz) ; 7,33 (dd, 4H, 2 x $H_{2'}$, $H_{6'}$, $J_{2',3'}$ = 8,6 Hz, $J_{2',F}$ = 5,44 Hz) ; 6,98 (t, 4H, 2 x $H_{3'}$, $H_{5'}$, $J_{3',2'}$ = $J_{3',F}$ = 8,6 Hz) ; 6,75-6,73 (m, 2H, $H_3$, $H_5$) ; 6,63-6,61 (m, 2H, $H_8$, $H_6$) ; 4,25 (s, 1H, N-**CH**-Ar) ; 3,69-3,63 (m, $4H_{pipérazine}$) ; 2,42-2,37 (m, $4H_{pipérazine}$).

En procédant comme pour l'exemple 53, on obtient également :

**EXEMPLE 55 : 6-BENZOYLOXY-2-(N-BUTYLANILINO)CARBONYL-1,4-BENZODIOXINE**

**EXEMPLE 56 : 6-(3,4,5-TRIMETHOXYBENZOYLOXY)-2-(N-BUTYLANILINO)CARBONYL-1,4-BENZODIOXINE**

**EXEMPLE 57 : 6-PROPANOYLOXY-2-{4-[BIS-4-FLUOROPHENYL)METHYL]PIPERAZIN-1-YL}CARBONYL-1,4-BENZODIOXINE**

**EXEMPLE 58 : 6-HYDROXY-2-{4-[BIS-(4-FLUOROPHENYL)METHYL]PIPERAZIN-1-YL}METHYL-1,4-BENZODIOXINE**

Chauffer à reflux pendant 3 heures sous atmosphère d'azote et en présence de 5 mmol d'hydrure double de lithium et d'aluminium une solution de 2 mmol de 6-hydroxy-2-{4-[bis-(4-fluorophényl)méthyl]pipérazin-1-yl}carbonyl-1,4-benzodioxine dans 35 $cm^3$ d'éther éthylique. Après refroidissement, hydrolyse, élimination par filtration des composés insolubles minéraux, et séchage sur sulfate de magnésium, le composé obtenu est purifié par chromatographie sur colonne de silice (éluant : éther de pétrole/acétate d'éthyle, 40:60). On obtient ainsi la 6-hydroxy-2-{4-[bis-(4-fluorophényl)méthyl]pipérazin-1-yl}méthyl-1,4-benzodioxine avec un rendement de 85 %.

Point de fusion : 190°C.

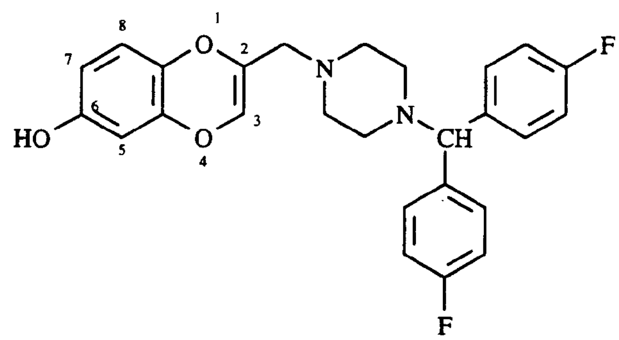

**$^1$H RMN ($CDCl_3$), δ (ppm) :** 9 (s, 1H, OH) ;7,34 (dd, 4H, 2 x $H_{2'}$, $H_{6'}$, $J_{2',3'}$ = 8,61 Hz, $J_{2',F}$ = 5,55 Hz) ; 6,96 (t, 4H, 2 x $H_{3'}$, $H_{5'}$, $J_{3',2'}$ = $J_{3',F}$ = 8,61 Hz) ; 6,48 (d, 1H, $H_8$, $J_{8,7}$ = 8,54 Hz) ; 6,23 (dd, 1H, $H_7$, $J_{7,8}$ = 8,54 Hz, $J_{7,5}$ = 2,87 Hz) ; 6,14 (d, 1H, $J_{5,7}$ = 2,87 Hz) ; 5,79 (s, 1H, $H_3$) ; 4,23 (s, 1H, NCHAr) ; 2,89 (s, 2H, $CH_2N$) ; 2,63-2,36 (m, $8H_{pipérazine}$).

**IR (KBr) ν ($cm^{-1}$) :** 3610-2985 (OH), 1695 (C = C), 1595-1495 (C = $C_{Ar}$), 1210-1180-1135-1075 (= C-O-C).

En procédant de la même façon, on obtient les composés suivants :

**EXEMPLE 59 : 7-HYDROXY-6-TERTIOBUTYL-2-{4-[BIS-(4-FLUOROPHENYL)METHYL]PIPERAZIN-1-YL}METHYL-2,3-DIHYDRO-1,4-BENZODIOXINE**

Ce composé est obtenu avec un rendement de 65 % à partir de la 7-hydroxy-6-tertiobutyl-2-{4-[bis-(4-fluorophényl)méthyl]pipérazin-1-yl}méthyl-2,3-dihydro-1,4-benzodioxine.

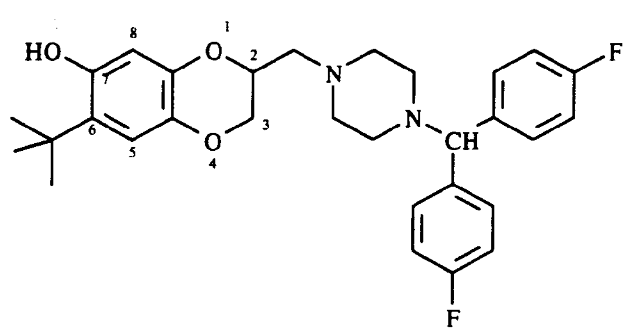

**$^1$H RMN ($CDCl_3$), δ (ppm) + $D_2O$ :** 7,32 (dd, 4H, 2 x $H_{2'}$, $H_{6'}$, $J_{2',3'}$ = 8,45 Hz, $J_{2',F}$ = 5,88 Hz) ; 6,96 (t, 4H,

2 x $H_{3'}$, $H_{5'}$, $J_{3',2'}$ = $J_{3',F}$ = 8,45 Hz) ; 6,75 (s, 1H, $H_5$) ; 6,26 (s, 1H, $H_8$) ; 4,41-4,29 (m, 1H, $H_2$) ; 4,18 (s, 1H, N-**CH**-Ar) ; 4,16 (dd, 1H, $H_{3b}$, $J_1$ = 11,4 Hz, $J_3$ = 1,47 Hz) ; 3,86 (dd, 1H, $H_{3b}$, $J_1$ = 11,4 Hz, $J_2$ = 6,62 Hz) ; 2,65-2,42 (m, 10H, $8H_{pipérazine}$ + $\mathbf{CH_2}$-N) ; 1,35 (s, 9H, $(\mathbf{CH_3})_3$C).

**EXEMPLE 60 : 7-HYDROXY-6-TERTIOBUTYL-2-{4-[BIS-(4-FLUOROPHENYL)METHYL]PIPERAZIN-1-YL}METHYL-1,4-BENZODIOXINE**

Ce composé est obtenu avec un rendement de 86 % à partir de la 7-hydroxy-6-tertiobutyl-2-{4-[bis-(4-fluorophényl)méthyl]pipérazin-1-yl}méthyl-1,4-benzodioxine.

Point de fusion : 186°C.

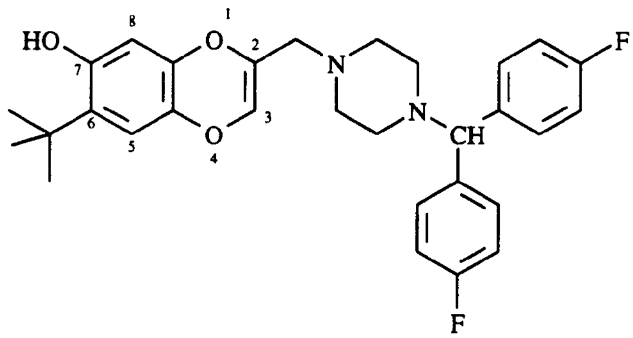

**$^1$H RMN ($CDCl_3$), δ (ppm)** : 7,33 (dd, 4H, 2 x $H_{2'}$, $H_{6'}$, $J_{2',3'}$ = 8,46 Hz, $J_{2',F}$ = 5,15 Hz) ; 6,98 (t, 4H, 2 x $H_{3'}$, $H_{5'}$, $J_{3',2'}$ = $J_{3',F}$ = 8,46 Hz) ; 6,56 (s, 1H, $H_3$) ; 6,45 (s, 1H, $H_5$) ; 6,23 (s, 1H, OH) ; 6,20 (s, 1H, $H_8$) ; 4,23 (s, 1H, N**CH**Ar) ; 3,67-3,60 (m, $4H_{pipérazine}$) ; 2,40-2,33 (m, 6H, $4H_{pipérazine}$ + $\mathbf{CH_2}$-N) ; 1,31 (s, 9H, $(\mathbf{CH_3})_3$C).

**IR (KBr) ν ($cm^{-1}$)** : 3610-3280-3100-3000 (OH), 1595-1495 (C = C).

**EXEMPLE 61 : 6-HYDROXY-7-TERTIOBUTYL-2-{4-[BIS-(4-FLUOROPHENYL)METHYL]PIPERAZIN-1-YL}METHYL-2,3-DIHYDRO-1,4-BENZODIOXINE**

Ce composé est obtenu avec un rendement de 73 % à partir de la 6-hydroxy-7-tertiobutyl-2-{4-[bis-(4-fluorophényl)méthyl]pipérazin-1-yl}méthyl-2,3-dihydro-1,4-benzodioxine.

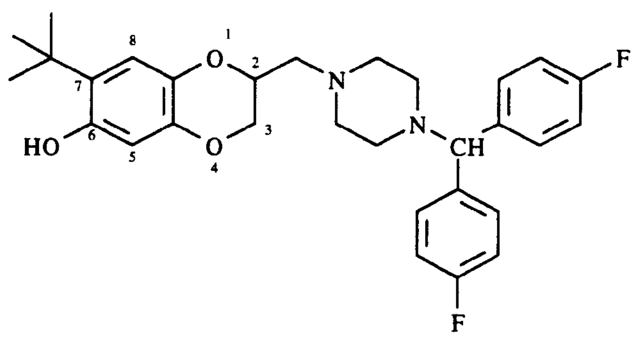

**$^1$H RMN ($CDCl_3$), δ (ppm)** : 7,33 (dd, 4H, 2 x $H_{2'}$, $H_{6'}$, $J_{2',3'}$ = 8,8 Hz, $J_{2',F}$ = 5,15 Hz) ; 6,96 (t, 4H, 2 x $H_{3'}$, $H_{5'}$, $J_{3',2'}$ = $J_{3',F}$ = 8,8 Hz) ; 6,76 (s, 1H, $H_8$) ; 6,24 (s, 1H, $H_5$) ; 4,28-4,15 (m, 3H, $H_2$, $H_{3a}$, N**CH**Ar) ; 2,7-2,51 (m, 6H, $4H_{pipérazine}$, $\mathbf{CH_2}$N) ; 2,5-2,2 (m, $4H_{pipérazine}$) ; 1,37 (s, 9H, $(\mathbf{CH_3})_3$C).

De la même façon, on obtient également :

**EXEMPLE 62 : 6-HYDROXY-2-(N-BUTYLANILINO)METHYL-1,4-BENZODIOXINE**

**EXEMPLE 63 : 6-HYDROXY-7-TERTIOBUTYL-2-(N-BUTYLANILINO)METHYL-1,4-BENZODIOXINE**

**EXEMPLE 64 : 6-HYDROXY-7-TERTIOBUTYL-2-(N-BUTYLAMINO)METHYL-2,3-DIHYDRO-1,4-BENZODIOXINE**

**EXEMPLE 65 : 6-METHOXY-2-(4-[BIS-(4-FLUOROPHENYL)METHYL]PIPERAZIN-1-YL}METHYL-1,4-BENZODIOXINE**

**EXEMPLE 66 : 6-HYDROXY-2-[(2,4-DIMETHYLPYRIDIN-6-YL)AMINO]METHYL-1,4-BENZODIOXINE**

**EXEMPLE 67 : 6-ETHYL-2-ANILINOMETHYL-1,4-BENZODIOXINE**

**EXEMPLE 68 : 6-HYDROXY-2-[4-(PYRIMID-2-YL)PIPERAZIN-1-YL]METHYL-1,4-BENZODIOXINE**

**EXEMPLE 69 : 7-HYDROXY-2-[4-(2,3,4-TRIMETHOXYBENZYL)PIPERAZIN-1-YL]METHYL-1,4-BENZODIOXINE**

**EXEMPLE 70 : 6-HYDROXY-7-TERTIOBUTYL-2-PIPERIDINOMETHYL-1,4-BENZODIOXINE**

**EXEMPLE 71 : 6-HYDROXY-2-(4-PHENYLPIPERIDINOMETHYL)-1,4-BENZODIOXINE**

**EXEMPLE 72 : 6-HYDROXY-2-{4[BIS-(4-FLUOROPHENYL)METHYLENE]PIPERIDINOMETHYL}-1,4-BENZODIOXINE**

**EXEMPLE 73 : 6-HYDROXY-2-(N,N-DIPROPYLAMINOMETHYL)-1,4-BENZODIOXINE**

**EXEMPLE 74 : 7-METHOXY-2-(N,N-DIPROPYLAMINOMETHYL)-2,3-DIHYDRO-1,4-BENZODIOXINE**

**EXEMPLE 75 : 7-HYDROXY-2-[4-(4-FLUOROPHENYL)PIPERAZIN-1-YL]METHYL-2,3-DIHYDRO-1,4-BENZODIOXINE**

**EXEMPLE 76 : 2-{4-[BIS-(4-FLUOROPHENYL)METHYL]PIPERAZIN-1-YL}THIOCARBONYL-1,4-BENZODIOXINE**

Additionner successivement 0,054 g (0,12 mmol) de pentaphosphore disulfure ($P_4S_{10}$) et 0,555 g (6,7 mmol) d'hydrogénocarbonate de sodium solide à 0,5 g (1,1 mmol) du composé obtenu dans l'exemple 9 en solution dans 8 ml de toluène et 2 ml d'acétonitrile, sous atmosphère inerte. Porter à reflux pendant 8 heures puis refroidir et rincer abondamment par du chlorure de méthylène. Après mise à sec sous pression réduite du milieu réactionnel, le produit brut est purifié par chromatographie sur colonne de silice (éluant : éther de pétrole/acétate d'éthyle, 70:30). Le composé attendu est obtenu avec un rendement de 80 %.

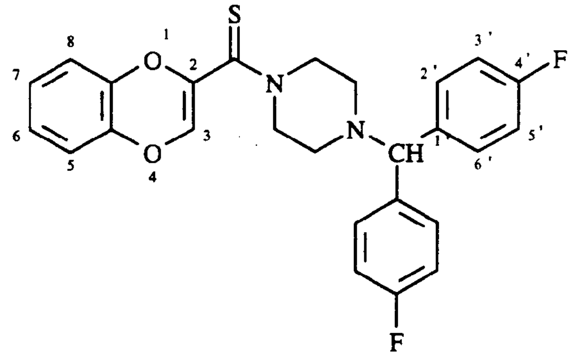

**$^1$H RMN ($CDCl_3$), δ (ppm)** : 7,38 (dd, 4H, 2 x $H_{2'}$, $H_{6'}$, $J_{2',3'}$ = 8,46 Hz, $J_{2',F}$ = 5,15 Hz) ; 7,01 (t, 4H, 2 x $H_{3'}$, $H_{5'}$, $J_{3',2'}$ = $J_{3',F}$ = 8,46 Hz) ; 6,89-6,58 (m, 5H, 4$H_{aromatique}$, $H_3$); 4,3 (s, 1H, NCHAr) ; 4,2-3,87 (m, 4H, $H_{pipérazine}$) ;

2,67-2,43 (m, $4H_{pipérazine}$).

**IR (KBr) ν ($cm^{-1}$) :** 1290 (C=S)

**EXEMPLE 77 : 6-HYDROXY-2-{4-[BIS(4-FLUOROPHENYL)-METHYL]PIPERAZIN-1-YL}THIOCARBONYL-1,4-BENZODIOXINE**

METHODE A

17 mmoles du composé obtenu dans l'exemple 1 sont dissoutes dans 125 $cm^3$ de toluène anhydre. 10,2 mmoles de réactif de Lawesson sont ajoutées, et l'ensemble est porté à reflux pendant 6 heures. Après évaporation du solvant et purification sur colonne de silice (éluant : dichlorométhane), le produit est obtenu avec un rendement de 12 %, sous forme d'huile.

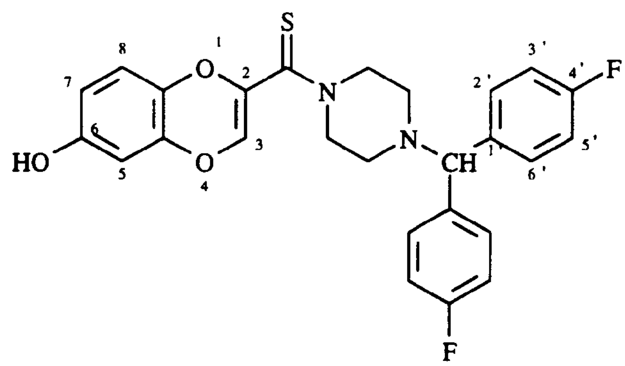

**$^1$H RMN ($CDCl_3$), δ (ppm)** : 7,36 (dd, 4H, 2 x $H_{2'}$, $H_{6'}$, $J_{2',3'}$ = 8,59 Hz, $J_{2',F}$ = 5,42 Hz) ; 7 (t, 4H, 2 x $H_{3'}$, $H_{5'}$, $J_{3',2'}$ = $J_{3',F}$ = 8,59 Hz) ; 6,6 (s, 1H, $H_3$) ; 6,48 (d, 1H, $H_8$, $J_{8,7}$ = 8,60 Hz) ; 6,30 (dd, 1H, $H_7$, $J_{7,5}$ = 2,89 Hz, $J_{7,8}$ = 8,60 Hz) ; 6,23 (d, 1H, $H_5$, $J_{5,7}$ = 2,89 Hz) ; 4,28 (s, 1H, NCHAr) ; 4,12-3,92 (m, $4H_{pipérazine}$) ; 3,45 (s, 1H, OH) ; 2,57-2,47 (m, $4H_{pipérazine}$).

**IR (KBr) ν ($cm^{-1}$) :** 3600-2980-2800 (OH); 1280 (C=S)

METHODE B

*Stade 1: 6-Acétoxy-2-{4-[bis(4-fluorophényl)méthyl]pipérazin-1-yl}carbonyl-1,4-benzodioxine*

Dissoudre sous atmosphère inerte à 0°C, 0,5 g (1,07 mmol) de composé obtenu dans l'exemple 1 dans 0,255 ml (2,7 mmol) d'anhydride acétique. Ajouter 2 volumes de pyridine (0,510 ml) et laisser sous agitation en ramenant à température ambiante pendant 1 h 30. Neutraliser avec une solution saturée d'hydrogénocarbonate de sodium. Après extraction à l'éther (2 x 25 ml), le produit est purifié par chromatographie sur colonne de silice (éluant : acétate d'éthyle/éther de pétrole, 20:80) et obtenu pur sous forme solide avec un rendement de 92 %.

Point de fusion : 69°C

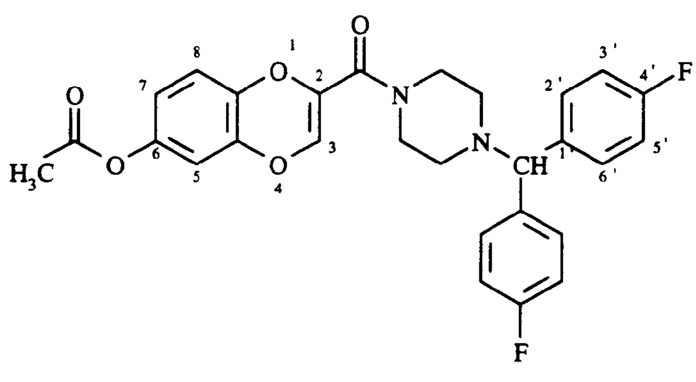

**$^1$H RMN ($CDCl_3$), δ (ppm)** : 7,35 (dd, 4H, 2 x $H_{2'}$, $H_{6'}$, $J_{2',3'}$ = 8,57 Hz, $J_{2',F}$ = 5,47 Hz) ; 6,93 (t, 4H, 2 x $H_{5'}$,

$H_{3'}$, $J_{3',2'}$ = $J_{3',F}$ = 8,57 Hz) ; 6,63-6,61 (m, 2H, $H_3$, $H_8$, $J_{8,7}$ = 8,39 Hz) ; 6,57 (dd, 1H, $H_7$, $J_{7,5}$ = 2,48 Hz, $J_{7,8}$ = 8,39 Hz) ; 6,48 (d, 1H,$H_5$, $J_{5,7}$ = 2,48 Hz) ; 4,25 (s, 1H, NCHAr) ; 3,7-3,60 (m, $4H_{pipérazine}$) ; 2,45-2,36 (m, $4H_{pipérazine}$) ; 2,25 (s, 3H, $CH_3COO$).

**IR (KBr) ν (Cm$^{-1}$) :**

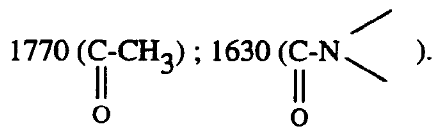

<u>Stade 2</u>: *6-Acétoxy-2-{4-[bis(4-fluorophényl)méthyl]pipérazin-1-yl}thiocarbonyl-1,4-benzodioxine*

Dissoudre 0,5 g (0,99 mmol) du composé obtenu à l'étape précédente dans 8 ml de toluène anhydre. Ajouter 0,49 g (0,99 mmol) de réactif de Lawesson et porter à reflux pendant 12 heures. Après mise à sec sous pression réduite du milieu réactionnel, le produit est purifié par chromatographie sur colonne de silice (éluant : éther de pétrole/acétate d'éthyle, 70:30). Le thioamide est obtenu avec un rendement de 78 %, sous forme de mousse.

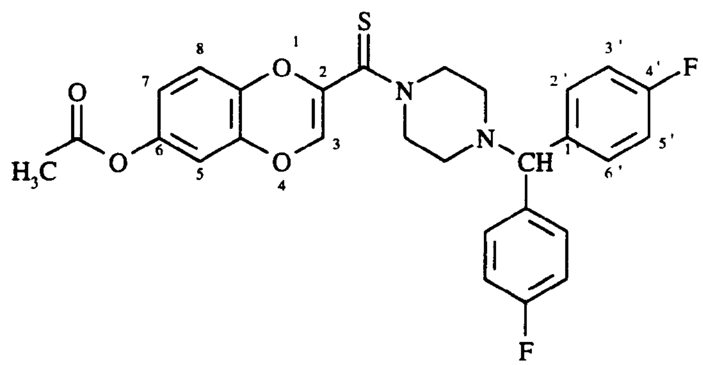

**<u>$^1$H RMN ($CDCl_3$), δ (ppm)</u>** : 7,36 (dd, 1H, 2 x $H_{2'}$, $H_{6'}$, $J_{2',F}$ = 5,41 Hz, $J_{2',3'}$ = 8,53 Hz) ; 7 (t, 2H, 2 x $H_{3'}$, $H_{5'}$, $J_{3',2'}$ = $J_{3',F}$ = 8,53 Hz) ; 6,65-6,6 (m, 2H, $H_3$, $H_8$, $J_{8,7}$ = 8,62 Hz) ; 6,57 (dd, 1H, $H_7$, $J_{7,5}$ = 2,47 Hz, $J_{7,8}$ = 8,62 Hz) ; 6,49 (d, 1H, $H_5$, $J_{5,7}$ = 2,47 Hz) ; 4,28 (s, 1H, NCHAr) ; 4,13-3,90 (m, $4H_{pipérazine}$) , 2,56-2,45 (m, $4H_{pipérazine}$) ; 2,24 (s, 3H, $CH_3COO$).

**IR (film) ν (Cm$^{-1}$):** 1765 (C=O) ; 1295 (C=S).

<u>Stade 3</u>: *6-Hydroxy-2-{4-[bis(4-fluorophényl)méthyl]pipérazin-1-yl}thiocarbonyl-1,4-benzodioxine*

Additionner 0,150 cm$^3$ d'une solution molaire de méthylate de sodium dans le méthanol à une solution de 0,5 g du composé thioamide acétoxylé précédent (0,96 mmol) dans 5 cm$^3$ de méthanol anhydre sous atmosphère d'azote. Agiter 3 heures à température ambiante puis neutraliser avec de la résine Dowex X-8, acide, préalablement lavée au méthanol. Après filtration et évaporation sous vide, le produit brut obtenu est purifié par chromatographie sur colonne de silice (éluant : éther de pétrole/acétate d'éthyle, 30:70). On obtient ainsi le thioamide phénolique avec un rendement de 90 %, sous forme d'huile.

<u>Rendement global des 3 étapes</u> : 64,5 %.

De la même façon, on obtient :

**<u>EXEMPLE 78</u> : 7-HYDROXY-6-TERTIOBUTYL-2-{4-[BIS(4-FLUOROPHENYL)METHYL]PIPERAZIN-1-YL}THIOCARBONYL-2,3-DIHYDRO-1,4-BENZODIOXINE**

Obtenu à partir du composé de l'exemple 5.

**<u>EXEMPLE 79</u> : 6-HYDROXY-2-(N-BUTYLANILINO)THIOCARBONYL-1,4-BENZODIOXINE**

Obtenu à partir du composé de l'exemple 14.

**EXEMPLE 80 : 7-HYDROXY-2-[4-(2,3,4-TRIMETHOXYBENZYL)PIPERAZIN-1-YL]THIOCARBONYL-1,4-BENZODIOXINE**

Obtenu à partir du composé de l'exemple 16.

**EXEMPLE 81 : 6-HYDROXY-2-(4-PHENYLPIPERIDINOTHIOCARBONYL)-1,4-BENZODIOXINE**

Obtenu à partir du composé de l'exemple 21.

**EXEMPLE 82 : 6-HYDROXY-2-[4-(4-FLUOROBENZOYL)PIPERIDINOTHIOCARBONYL]-1,4-BENZODIOXINE**

Obtenu à partir du composé de l'exemple 24.

**EXEMPLE 83 : 6-HYDROXY-2-(QUINOLEIN-3-YL)AMINOTHIOCARBONYL-1,4-BENZODIOXINE**

Obtenu à partir du composé de l'exemple 35.

**EXEMPLE 84 : 6-(3,4,5-TRIMETHOXYBENZOYLOXY)-2-(N-BUTYLANILINO)THIOCARBONYL-1,4-BENZODIOXINE**

Obtenu à partir du composé de l'exemple 56.

**EXEMPLE 85 : 6-NICOTINOYLOXY-2-{4-[BIS-(4-FLUOROPHENYL)METHYL]PIPERAZIN-1-YL}THIOCARBONYL-1,4-BENZODIOXINE**

Obtenu à partir du composé de l'exemple 53.

**ETUDE PHARMACOLOGIQUE**

**EXEMPLE A : Etude du pouvoir protecteur de l'oxydation des LDL (Méthode électrophorétique)**

La capacité des composés de l'invention à diminuer les proportions de LDL oxydées a été mesurée par incubation de 24 heures d'une préparation comprenant des LDL humaines natives, du $Cu^{2+}$ générateur de radicaux libres et les composés à tester. Les produits d'oxydation sont dosés par FPLC (Fast Protein Liquid Chromatography) selon la méthode décrite par B. Vedie *et al.*, Journal of Lipid Research, 32, 1359-1369, (1991). Cette méthode permet d'identifier 5 pics correspondant aux différents degrés d'oxydation des LDL. Les pics A et B correspondent aux formes natives des LDL et les pics C, D et E correspondent aux différents états d'oxydation des LDL (le pic E correspondant à la forme la plus oxydée). Les résultats sont exprimés en pourcentage des LDL correspondant à ces différents états d'oxydation.

Un effet protecteur d'un composé vis-à-vis de l'oxydation induite par le cuivre se traduit par un déplacement de la forme E vers la forme D voire vers la forme C ou la forme B pour les composés les plus puissants.

**- Tableau I -**

| - Pouvoir protecteur de l'oxydation des LDL - | | | | | |
|---|---|---|---|---|---|
| **FORMES OXYDEES SEPAREES PAR FPLC** | **A** | **B** | **C** | **D** | **E** |
| Témoin sans $Cu^{++}$ | | 100 % | | | |
| Témoin avec $Cu^{++}$ | | | | 75 % | 25 % |
| $Cu^{++}$ + Probucol (10 μM) | | | | 80 % | 20 % |
| $Cu^{++}$ + composé de l'exemple 1 (10 μM) | | 95 % | 5 % | | |

Les composés de l'invention sont remarquablement actifs dans ce test avec des activités très supérieures à celles constatées pour le Probucol utilisé comme référence.

**EXEMPLE B : Etude du pouvoir protecteur de l'oxydation des LDL (dosage du malondialdéhyde)**

Des LDL humaines purifiées sont incubées en présence de sulfate de cuivre à la concentration de $5.10^{-6}$ M en l'absence ou en présence des composés à étudier.

L'activité des produits testés est évaluée par le calcul de la concentration réduisant de 50 % ($IC_{50}$) la production de malondialdéhyde (MDA) par rapport aux expériences contrôles effectuées en l'absence de produit. Les composés de l'invention sont remarquablement actifs dans ce test avec des $IC_{50}$ de l'ordre de $10^{-7}$ M très largement inférieures à celles déterminées pour le Probucol.

**EXEMPLE C : Mesure de l'hémolyse induite par l'AAPH**

Des hématies humaines sont placées en présence d'AAPH, générateur de radicaux libres à taux constant et en présence ou en absence des composés à étudier durant 30 minutes à 37°C. La densité optique du surnageant est mesurée à 403 nm par rapport au témoin sans AAPH. Le pourcentage d'inhibition de l'hémolyse est calculé par comparaison aux 100 % d'hémolyse obtenus pour le témoin AAPH.

**- Tableau II -**

| - % d'inhibition à $10^{-5}$M de l'hémolyse AAPH - | | | | | | | | |
|---|---|---|---|---|---|---|---|---|
| **EXEMPLE** | **1** | **2** | **3** | **10** | **11** | **12** | **13** | **17** |
| Inhibition | 62 % | 100 % | 88 % | 100 % | 100 % | 100 % | 100 % | 100 % |

Les composés selon l'invention montrent donc une remarquable activité protectrice cellulaire vis à vis de la toxicité radicalaire induite par l'AAPH.

**EXEMPLE D : Mise en évidence de l'activité anti-hypoxique in vivo**

Des souris mâles (Swiss CD1) pesant de 25 à 30 g sont stabulées durant une semaine avant toute expérience dans les conditions usuelles d'animalerie (20-22°C, 55 % d'humidité, cycle lumière / obscurité 12/12, nourriture industrielle et eau à volonté). Les souris sont placées dans une boîte (7 x 5 x 5 cm) dans laquelle on crée une atmosphère pauvre en oxygène par le passage d'un courant d'air (96 % $N_2$, 4 % $O_2$, 12 l/mn). Le délai d'apparition des premières suffocations (ou "gasps") est mesuré. Les souris recoivent une dose des composés à tester par voie intrapéritonéale 30 mn avant la réalisation de l'hypoxie. La vincamine est utilisée comme drogue de référence.

**- Tableau III -**

**- Mise en évidence de l'activité anti-hypoxique -**

| **TRAITEMENT** | **DOSE** (mg/kg) | **DELAI D'APPARITION DES PREMIER "GASPS"** (en secondes) |
|---|---|---|
| Témoin | - | 36 ± 4 |
| Vincamine | 2,5 | inactif |
| Vincamine | 20 | 147 ± 14 (significatif) |
| Exemple 1 | 2,5 | 108 (significatif) |
| Exemple 10 | 2,5 | 97 (significatif) |

Les résultats montrent que les composés de l'invention sont significativement actifs dans ce test à des

doses très inférieures à celles de la vincamine (produit de référence).

**EXEMPLE E : Mesure des activités lipoxygénase**

Les mesures sont réalisées sur des granulocytes de lapins. Les granulocytes isolés sont stimulés *in vitro* par l'ionophore calcique A 23187. Les leukotriènes B4 libérés dans le milieu extracellulaire sont mesurés par Radio Immuno Assay (RIA). Les résultats (moyenne des 3 mesures indépendantes) sont exprimés en pourcentage d'inhibition par rapport au témoin.

Dans cette étude, le composé de l'exemple 1 (10 μM) présente un pouvoir inhibiteur de 62 %.

**EXEMPLE F : Mesure de l'activité antiagrégante**

Dans cette étude, les composés de l'invention (100 μg/ml) ont été testés dans le but de quantifier leur pouvoir d'inhibition du maximum non-réversible d'agrégation plaquettaire (plasma de lapin enrichi en plaquettes) induite par l'arachidonate de sodium (50 μg/ml). Ainsi, la concentration minimale active pour le composé de l'exemple 1 est de 20 μM.

**EXEMPLE G : Activité anticalcique**

L'activité anticalcique a été étudiée sur l'aorte thoracique de rat mâle Wistar. Après leur installation dans les cuves expérimentales contenant une solution physiologique, les anneaux vasculaires sont soumis à une tension initiale de 2 g. Les préparations sont ensuite mises en présence d'une solution hyperpotassique (60 mM de chlorure de potassium) pour l'obtention d'une réponse contractile soutenue. Des concentrations croissantes de produit à tester sont alors ajoutées pendant le temps nécessaire à l'obtention d'un effet stable.

Chaque composé de l'invention est ainsi testé sur 2 préparations à 8 concentrations ($10^{-8}$ M à $3.10^{-5}$M) pour l'obtention d'une courbe effet-concentration et la détermination de l'$IC_{50}$.

**- Tableau IV -**

| - Inhibition de la concentration de l'aorte thoracique du rat induite par le chlorure de potassium - | |
|---|---|
| **Exemple** | **$IC_{50}$ (μM)** |
| 1 | 0,5 |
| 58 | 0,6 |

**EXEMPLE H : Activité hypolipémiante**

Des groupes de 6 souris sont rendus hypercholestérolémiques par un régime alimentaire élevé en cholestérol et en acide cholique pendant 7 jours. Les composés de l'invention sont administrés p.o. (100 mg/kg) le 6ème et le 7ème jour, (une demi-dose étant administrée le 6ème jour, l'autre demi-dose étant administrée le 7ème jour). Les animaux sont ensuite mis à jeûn pendant une nuit. La diminution de la concentration en cholestérol dans le sérum par rapport à des souris hypercholestérolémiques contrôles est alors évaluée, de même que la diminution des concentrations en lipoprotéines (correspondant au fractions LDL-VLDL), après précipitation par addition d'héparine dans le sérum, sur les mêmes souris hypercholestérolémiées par rapport aux animaux contrôles.

Le composé de l'exemple 58 a provoqué une baisse significative du cholestérol total (33 % à 100 mg/kg p.o.) et une diminution significative de la fraction LDL-VLDL (35 % à 100 mg/kg p.o.).

**EXEMPLE I : Activité sérotoninergique**

Les expériences sont réalisées sur des membranes préparées à partir du cortex cérébral de rats mâles Wistar (CERJ, le Genest St Isle). Les mesures de binding sont effectuées en utilisant la kétansérine, comme

composé de référence. Les conditions expérimentales sont résumées ci-dessous :

| Récepteur | Ligand | Concentrations | non spécifiques | Incubation |
|---|---|---|---|---|
| 5-$HT_2$ | [$^3$H]Kétansérine | 0,5 nM | Kétansérine (1μM) | 40mn/25°C |

Après incubation, les membranes sont rapidement filtrées. les filtrats sont ensuite transférés dans des fioles contenant du liquide scintillant et la radioactivité fixée est déterminée par un compteur à scintillation liquide (LS6000, Beckman). L'affinité des composés de l'invention pour les récepteurs 5-$HT_2$ est évaluée, à la concentration de 10 μM (n = 3), à partir d'expériences de compétition. Dans ces conditions, le composé de l'exemple 58 a montré un pouvoir d'inhibition de 79 %.

**<u>EXEMPLE J</u> : Composition pharmaceutique : comprimés**

Formule de préparation pour 1000 comprimés dosés à 50 mg.

| | |
|---|---|
| Composé de l'exemple 1 | 50 g |
| Amidon de blé | 15 g |
| Amidon de maïs | 15 g |
| Lactose | 65 g |
| Stéarate de magnésium | 2 g |
| Silice | 1 g |
| Hydroxypropylcellulose | 2 g |

## Revendications

1. Composés de formule générale (I) :

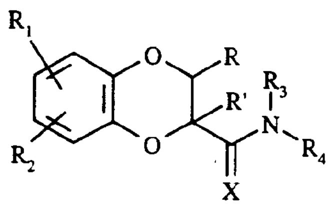

(I)

dans laquelle :

- R et R' représentent chacun un atome d'hydrogène, cas des 2,3-dihydrobenzodioxines, ou forment ensemble une liaison, cas des benzodioxines,
- $R_1$ et $R_2$ identiques ou différents sont choisis indépendamment l'un de l'autre parmi un atome d'hydrogène, un radical alkyle, contenant de 1 à 6 atomes de carbone en chaîne droite ou ramifiée, un radical hydroxy, un radical alkoxy contenant de 1 à 5 atomes de carbone en chaîne droite ou ramifiée, un groupement :

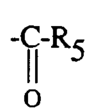 , et un groupement 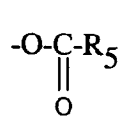 ,

dans lesquels $R_5$ est choisi parmi un radical alkyle contenant de 1 à 5 atomes de carbone en chaîne droite ou ramifiée, un radical aryle éventuellement substitué choisi parmi les radicaux phényle et

naphtyle, un radical arylalkyle, éventuellement substitué, choisi parmi les radicaux phényle et naphtyle fixés sur une chaîne alkyle comportant de 1 à 4 atomes de carbone, un radical hétéroaryle, éventuellement substitué, choisi parmi les radicaux pyridyle, pyrrolyle, pyrazinyle, pyridazinyle, pyrimidinyle, quinolyle, quinazolinyle et indolyle, et un radical hétéroarylalkyle, éventuellement substitué, choisi parmi les radicaux hétéroaryles ci-dessus définis fixés sur une chaîne alkyle comportant de 1 à 4 atomes de carbone,

- $R_3$ et $R_4$, identiques ou différents, sont choisis indépendamment l'un de l'autre parmi un atome d'hydrogène, un radical alkyle contenant de 1 à 5 atomes de carbone en chaîne droite ou ramifiée et éventuellement substitué, un radical aryle, arylalkyle, hétéroaryle et hétéroarylalkyle, chacun pouvant être éventuellement substitué par une ou plusieurs entités chimiques choisies parmi les radicaux définis pour $R_1$ ou $R_2$,

ou $R_3$ et $R_4$ forment ensemble avec l'atome d'azote auquel ils sont reliés un hétérocycle de formule (I') :

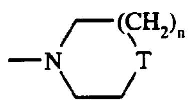

(I')

dans laquelle,

- n est choisi parmi 0, 1, 2 et 3,
- T est choisi parmi l'oxygène, le groupement

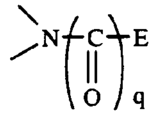

,

- le groupement

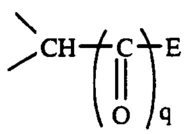

,

- et le groupement

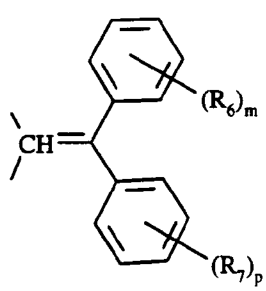

,

dans lequel :

m et p identiques ou différents prennent indépendamment les valeurs 0, 1, 2, 3, 4 ou 5,

$R_6$ et $R_7$ identiques ou différents, indépendamment l'un de l'autre, sont choisis parmi un halogène, un groupement hydroxy, alkyle linéaire ou ramifié comprenant de 1 à 5 atomes de carbone, un radical alkoxy, un radical halogénoalkyle et un radical polyhalogénoalkyle,

- q est choisi parmi 0 et 1,
- E est choisi parmi un atome d'hydrogène, un radical alkyle contenant de 1 à 5 atomes de carbone en chaîne droite ou ramifiée, un radical aryle, arylalkyle, hétéroaryle, hétéroarylalkyle, tels que définis précédemment, chacun éventuellement substitué par un ou plusieurs radicaux $R_6$ tel que

définis précédemment et le groupement :

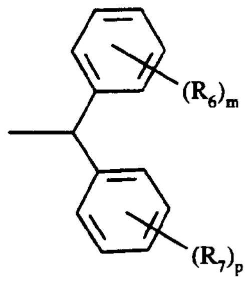

,

dans lequel $R_6$, $R_7$, m et p sont tels que définis précédemment,

avec la restriction lorsque X = 0 et $R_1$ = $R_2$ = H alors T ne peut représenter un radical $CH_2$ ou un radical quinazolinyl-2-ylamino,

- et X est choisi parmi O, S ou $H_2$,

étant entendu que, sauf indication contraire, le terme "éventuellement substitué" indique une substitution éventuelle par un ou plusieurs radicaux choisis parmi les radicaux hydroxy, nitro, cyano, alkyles, alkoxy, acyles, halogénoalkyles, polyhalogénoakyles, amino, alkylamino et dialkylamino, les chaînes alkyles des groupements alkyles, alkoxy, acyles, halogénoalkyles, polyhalogénoalkyles, alkylamino et dialkylamino comportant de 1 à 5 atomes de carbone en chaîne droite ou ramifiée,

avec les restrictions suivantes :

- lorsque R et R' représentent chacun l'hydrogène, alors X ne peut représenter $H_2$,
- lorsque R et R' représentent chacun l'hydrogène et X représente l'oxygène, alors
  - soit $R_1$ et $R_2$ sont chacun différents de l'hydrogène,
  - soit $R_1$ est différent de l'hydrogène et $R_3$ et $R_4$ forment ensemble, avec l'atome d'azote auquel ils sont reliés, un hétérocyle tel que défini précédemment,

leurs éventuels stéréoisomères, N-oxydes et sels d'addition à un acide ou à une base, pharmaceutiquement acceptables.

**2.** Composés selon la revendication 1, de formule (I) dans laquelle X représente l'oxygène, leurs éventuels stéréoisomères, N-oxydes et sels d'addition à un acide ou à une base, pharmaceutiquement acceptables.

**3.** Composés selon la revendication 1, de formule (I) dans laquelle X représente le soufre, leurs éventuels stéréoisomères, N-oxydes et sels d'addition à un acide ou à une base, pharmaceutiquement acceptables.

**4.** Composés selon la revendication 1, de formule (I) dans laquelle X représente $H_2$, leurs éventuels stéréoisomères, N-oxydes et sels d'addition à un acide ou à une base, pharmaceutiquement acceptables.

**5.** Composés selon la revendication 1 pour lesquels les radicaux $R_3$ et $R_4$ forment avec l'atome d'azote auquel ils sont liés un radical pipérazinyle substitué, leurs éventuels stéréoisomères, N-oxydes et sels d'addition à un acide ou à une base, pharmaceutiquement acceptables.

**6.** Composé selon la revendication 1 qui est la 6-hydroxy-2-{4-[bis-(4-fluorophényl)méthyl]pipérazin-1-yl}carbonyl-1,4-benzodioxine ainsi que ses sels d'addition à un acide ou à une base, pharmaceutiquement acceptables.

**7.** Composé selon la revendication 1 qui est la 6-hydroxy-7-tertiobutyl-2-{4-[bis-(4-fluorophényl)méthyl]pipérazin-1-yl)carbonyl-1,4-benzodioxine ainsi que ses sels d'addition à un acide ou à une base, pharmaceutiquement acceptables.

**8.** Composé selon la revendication 1 qui est la 6-hydroxy-2-{4-[bis-(4-fluorophényl)méthyl]pipérazin-1-yl)méthyl-1,4-benzodioxine ainsi que ses sels d'addition à un acide, pharmaceutiquement acceptables.

**9.** Composé selon la revendication 1 qui est la 6-hydroxy-2-(N-butylanilino)carbonyl-1,4-benzodioxine ainsi que ses sels d'addition à un acide ou à une base, pharmaceutiquement acceptables.

**10.** Procédé de préparation des composés de formule (I), caractérisé en ce que l'on effectue une réaction de cyclisation en présence de carbonate alcalin, en solvant aprotique, à reflux, à partir de catéchol et de

2,3-dibromopropionate d'alkyle, conduisant au composé de formule (IIa) :

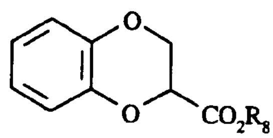

(IIa)

dans laquelle $R_8$ représente un radical alkyle comprenant de 1 à 5 atomes de carbone en chaîne droite ou ramifiée,
qui est éventuellement engagé dans une réaction d'acylation avec un acide de Lewis et un chlorure d'acyle de formule (III) :

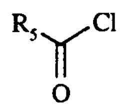

(III)

dans laquelle $R_5$ est tel que défini précédemment,
pour conduire au composé de formule (IIb) :

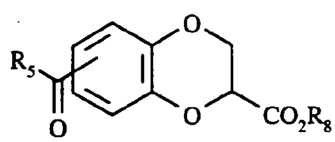

(IIb)

dans laquelle $R_5$ et $R_8$ sont tels que définis précédemment,
qui peut être éventuellement oxydé en diester correspondant de formule (IIc) :

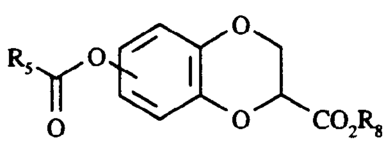

(IIc)

dans laquelle $R_5$ et $R_8$ sont tels que définis précédemment,
au moyen d'un agent d'oxydation, puis éventuellement transformé, par action d'un alcoolate alcalin, en phénol de formule (IId) :

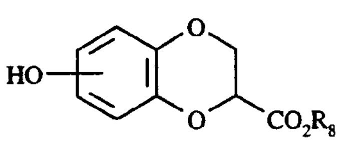

(IId)

dans laquelle $R_8$ est tel que défini précédemment,
les céto-esters de formule (IIb) pouvant d'autre part être hydrogénés, par action d'hydrogène et de palladium, en composé de formule (IIe) :

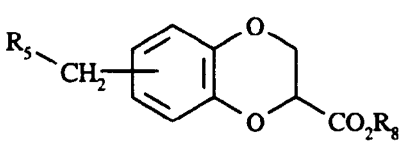

(IIe)

dans laquelle $R_5$ et $R_8$ sont tels que définis précédemment,

le phénol de formule (IId) pouvant être éthérifié par réaction avec un composé halogéné de formule (IV) :

$R_5$-Hal (IV)

dans laquelle Hal est un halogène,

pour conduire au composé de formule (IIf) :

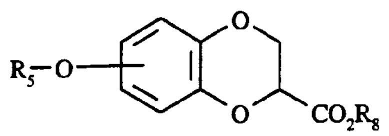

(IIf)

dans laquelle $R_5$ et $R_8$ sont tels que définis précédemment,

ou acylé par un chlorure d'acyle de formule (III) comme défini précédemment pour accéder au diester de formule (IIc) précédemment défini,

les composés de formules (IIa) à (IIf) formant l'ensemble des composés de formule (II) :

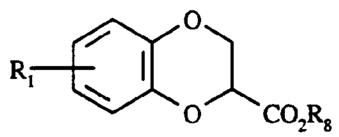

(II)

dans laquelle $R_1$ et $R_8$ sont tels que définis précédemment,

qui peuvent être à nouveau traités comme les composés de formules (IIa) à (IIf) ou en milieu acide par un alcool tertiaire de formule (X) :

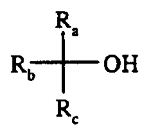

(X)

dans laquelle Ra, Rb et Rc représentent indépendamment un radical alkyle linéaire ou ramifié contenant de 1 à 3 atomes de carbone,

pour donner les composés de formule (Va) :

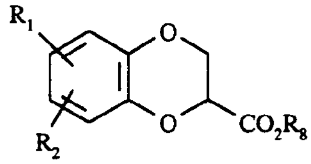

(Va)

dans laquelle $R_1$, $R_2$ et $R_8$ sont tels que définis précédemment,

les benzodioxanes de formules (Va) pouvant être éventuellement traitées par un agent bromant, en présence d'un initiateur radicalaire, pour conduire à l'intermédiaire de formule (VI) :

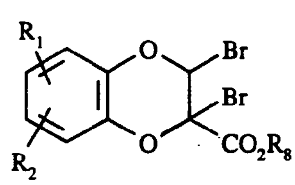

(VI)

dans laquelle $R_1$, $R_2$ et $R_8$ sont tels que définis précédemment,

qui est ensuite soumis à un halogénure alcalin, pour fournir les benzodioxines correspondantes de formule (Vb) :

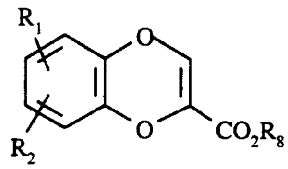

(Vb)

dans laquelle $R_1$, $R_2$ et $R_8$ sont tels que définis précédemment,
les composés de formules (Va) et (Vb) formant l'ensemble des composés de formule (V) :

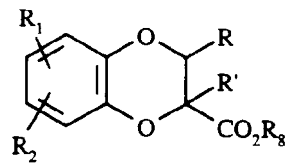

(V)

dans laquelle $R_1$, $R_2$, $R_8$, R et R', sont tels que définis précédemment,
qui sont finalement saponifiés pour conduire aux acides de formule (VII) :

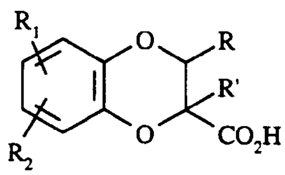

(VII)

dans laquelle R, R', $R_1$ et $R_2$ sont tels que définis précédemment,
qui sont soumis à une réaction de couplage peptidique, en présence de (1-(3-diméthylaminopropyl)-3-éthylcarbodiimide (EDCI) et d'hydroxybenzotriazole (HOBt) dans le diméthylformamide à 0°C, avec une amine de formule (VIII) :

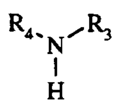

(VIII)

dans laquelle $R_3$ et $R_4$ sont tels que définis précédemment,
réaction conduisant aux composés de formule (IXa) :

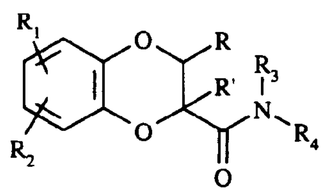

(IXa)

dans laquelle $R_1$, $R_2$, $R_3$, $R_4$, R et R' sont tels que définis précédemment,
cas particulier des composés de formule (I) dans laquelle X représente l'oxygène, qui sont :

- soit transformés par traitement de l'amide correspondant par le réactif de Lawesson, en thioamide de formule (IXb) :

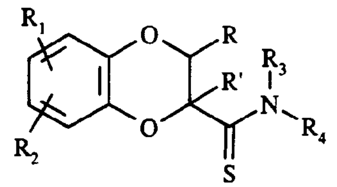

(IXb)

dans laquelle $R_1$, $R_2$, $R_3$, $R_4$, R et R' sont tels que définis précédemment,

cas particulier des composés de formule (I) dans laquelle X représente le soufre,

- soit transformés par réduction de l'amide correspondant grâce à un agent réducteur en solvant aprotique anhydre, en composés de formule (IXc) :

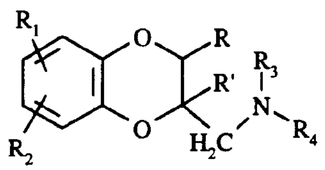

(IXc)

dans laquelle $R_1$, $R_2$, $R_3$, $R_4$, R et R' sont tels que définis précédemment,

cas particulier des composés de formule (I) dans laquelle X représente le groupement $H_2$,

l'ensemble des composés de formules (IXa), (IXb) et (IXc) formant l'ensemble des composés de formule (I) qui sont purifiés et éventuellement séparés en leurs stéréoisomères par une technique classique de séparation et qui sont, si on le désire, transformés en leurs sels d'addition à un acide ou à une base pharmaceutiquement acceptables.

**11.** Composés de formule (XI) utiles comme intermédiaires dans la préparation des composés de formule (I) :

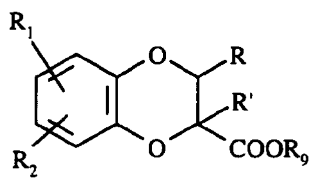

(XI)

dans laquelle :

- R et R' représentent chacun un atome d'hydrogène ou forment ensemble une liaison,
- $R_1$ et $R_2$ identiques ou différents sont choisis indépendamment l'un de l'autre parmi un atome d'hydrogène, un radical alkyle, contenant de 1 à 5 atomes de carbone en chaîne droite ou ramifiée, un radical hydroxy, un radical alkoxy contenant de 1 à 5 atomes de carbone en chaîne droite ou ramifiée, un groupement

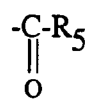 , et un groupement 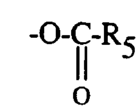 ,

dans lesquels $R_5$ est choisi parmi un radical alkyle contenant de 1 à 5 atomes de carbone en chaîne droite ou ramifiée, un radical aryle éventuellement substitué choisi parmi les radicaux phényle et naphtyle, un radical arylalkyle, éventuellement substitué, choisi parmi les radicaux phényle et naphtyle fixés sur une chaîne alkyle comportant de 1 à 4 atomes de carbone, un radical hétéroaryle, éventuellement substitué, choisi parmi les radicaux pyridyle, pyrrolyle, pyrazinyle, pyridazinyle, pyrimidinyle, quinolyle et indolyle et un radical hétéroarylalkle, éventuellement substitué, choisi parmi les radicaux hétéroaryles ci-dessus définis fixés sur une chaîne alkyle comportant de 1 à 4 atomes de carbone,

- $R_9$ est choisi parmi un atome d'hydrogène et un radical alkyle contenant de 1 à 5 atomes de carbone

en chaîne droite ou ramifiée,
avec les restrictions suivantes :

- lorsque $R_2$, R et R' représentent chacun l'hydrogène et $R_9$ un radical alkyle, alors $R_1$ ne peut représenter ni l'hydrogène, ni le radical méthoxy, ni le radical 1-hydroxyéthyle,
- lorsque $R_1$ et $R_2$ représentent chacun l'hydrogène et R et R' forment ensemble une liaison, alors $R_9$ ne peut représenter le radical éthyle,

ainsi que leurs éventuels stéréoisomères.

**12.** Compositions pharmaceutiques contenant comme principe actif au moins un composé selon l'une quelconque des revendications 1 à 9, seul ou en combinaison avec un ou plusieurs véhicules inertes, non toxiques et pharmaceutiquement acceptables.

**13.** Compositions pharmaceutiques selon la revendication 12 exerçant une activité anti-oxydante et protectrice dans les processus de peroxydation des lipides et utiles dans le traitement ou la prévention des affections où interviennent des processus oxydatifs et notamment les désordres ischémiques, les maladies métaboliques, l'athérome, l'artériosclérose, la protection cérébro- et cardiovasculaire ainsi que dans le traitement ou la prévention des dommages dus aux traumatismes chirurgicaux et à la reperfusion d'organes.

Office européen des brevets

# RAPPORT DE RECHERCHE EUROPEENNE

Numero de la demande
EP 94 40 0977

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.5) |
|---|---|---|---|
| X | GB-A-2 007 656 (PFIZER)<br>* le document en entier * | 1,2,12, 13 | C07D319/20<br>C07D405/12<br>A61K31/335 |
| A | FR-M-1 843 (GEIGY)<br>* le document en entier * | 1,10,12, 13 | |
| A | US-A-3 117 978 (J. BIEL ET AL)<br>* colonne 1 - colonne 4 * | 1,10,12, 13 | |
| A | US-A-2 366 611 (A. GRUN)<br>* le document en entier * | 1,12,13 | |
| A | EP-A-0 018 675 (SHELL)<br>* page 1 - page 8 * | 11 | |

DOMAINES TECHNIQUES RECHERCHES (Int.Cl.5)

C07D

Le présent rapport a été établi pour toutes les revendications

| Lieu de la recherche | Date d'achèvement de la recherche | Examinateur |
|---|---|---|
| LA HAYE | 17 Aoüt 1994 | Francois, J |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)